(19) Europäisches Patentamt
European Patent Office
Office européen des brevets

(11) **EP 4 052 772 A1**

(12) **EUROPEAN PATENT APPLICATION**
published in accordance with Art. 153(4) EPC

(43) Date of publication:
**07.09.2022 Bulletin 2022/36**

(21) Application number: **20883651.0**

(22) Date of filing: **30.10.2020**

(51) International Patent Classification (IPC):
*B01D 9/02* (2006.01)    *C07H 15/256* (2006.01)
*A23L 27/00* (2016.01)    *A23L 2/60* (2006.01)

(52) Cooperative Patent Classification (CPC):
**A23L 2/60; A23L 27/00; B01D 9/02; C07H 15/256**

(86) International application number:
**PCT/JP2020/040975**

(87) International publication number:
**WO 2021/085644 (06.05.2021 Gazette 2021/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **01.11.2019 JP 2019200257**

(71) Applicant: **Suntory Holdings Limited
Osaka 530-8203 (JP)**

(72) Inventors:
• **URAI, Soichiro
Kawasaki-shi, Kanagawa 211-0067 (JP)**
• **UTSUMI, Yui
Kawasaki-shi, Kanagawa 211-0067 (JP)**
• **TAKAYANAGI, Keisuke
Kawasaki-shi, Kanagawa 211-0067 (JP)**
• **NISHIBORI, Tomoyuki
Kawasaki-shi, Kanagawa 211-0067 (JP)**
• **MITSUI, Ryoki
Kawasaki-shi, Kanagawa 211-0067 (JP)**
• **YOKOO, Yoshiaki
Kawasaki-shi, Kanagawa 211-0067 (JP)**
• **NAGAO, Koji
Kawasaki-shi, Kanagawa 211-0067 (JP)**

(74) Representative: **Mewburn Ellis LLP
Aurora Building
Counterslip
Bristol BS1 6BX (GB)**

(54) **METHOD FOR MANUFACTURING REVAUDIOSIDE-D-CONTAINING CRYSTALLIZED PRODUCT, AND REVAUDIOSIDE-D-CONTAINING CRYSTALLIZED PRODUCT**

(57)    There is demand for a novel method for manufacturing a RebD crystallized product. The present invention provides a method for manufacturing a RebD-containing crystallized product, characterized in including: a step for mixing a stevia plant-derived crude product having a total steviol glycoside content of 50-95 mass%, and containing at least RebA and RebD, in a solvent containing ethanol and having a methanol concentration of 1 mg/L or less, and adjusting a crystallization solution; and a step for cooling the crystallization solution under stirring and causing RebD to precipitate.

**Description**

Technical Field

The present invention relates to a method for producing a rebaudioside D-containing crystallized product, in which a rebaudioside D-containing crystallized product having a high yield ratio of rebaudioside D and containing rebaudioside D at a high purity can be obtained, and a rebaudioside D-containing crystallized product.

Background Art

Leaves of Stevia rebaudiana of the Asteraceae family contain a secondary metabolite called steviol which is one of diterpenoids. Steviol glycosides exhibit sweetness about 300 times that of sugar, and thus are utilized as sweeteners with less calories in the food industry. Obesity is a serious social problem internationally, and sweeteners with less calories are increasingly demanded day by day also from the viewpoints of health promotion and a reduction in medical cost. Aspartame and acesulfame potassium, which are amino acid derivatives artificially synthesized, are currently utilized as artificial sweeteners. However, naturally occurring sweeteners with less calories, like steviol glycosides, are safer and expected to easily obtain the public acceptance.
There have been heretofore some reports about purification of steviol glycosides. For example, Patent Literature 1 discloses a method for purifying rebaudioside D by dissolving an extract of a stevia-rebaudiana-Bertoni plant in an aqueous solution in an organic solvent and performing crystallization three times or more. Patent Literature 2 discloses a method involving preparing a starting material including at least one steviol glycoside of a stevia-rebaudiana plant and an aqueous alcohol solution, dissolving the starting material in the aqueous alcohol solution and retaining the solution at about 24 to 80°C for about 1 minute to about 240 hours, and subjecting the resultant to solid-liquid separation and drying to thereby obtain a steviol glycoside composition, in which a steviol glycoside composition has a solubility of at least 0.01% in water at 20°C.

Citation List

Patent Literatures

Patent Literature 1: Japanese Patent Application Publication No. 2013-507914
Patent Literature 2: Japanese Patent Application Publication No. 2019-518065

Summary of Invention

Technical Problem

In the above circumstances, a novel method for producing a RebD crystallized product is currently demanded.

Solution to Problem

One aspect of the present invention provides the following.

[1] A method for producing a rebaudioside D-containing crystallized product by use of a roughly purified product obtained by roughly purifying an extract from a stevia plant, wherein

a total steviol glycoside content of the roughly purified product is 50 to 95% by mass and the roughly purified product contains at least rebaudioside A and rebaudioside D,
the method comprising:

a step of mixing the roughly purified product in a solvent containing ethanol and having a methanol concentration of 1 mg/L or less, to adjust a solution for crystallization, and
a step of cooling the solution for crystallization with stirring, to precipitate rebaudioside D.

[2] The production method according to [1], wherein the total steviol glycoside corresponds to rebaudioside A, rebaudioside B, rebaudioside C, rebaudioside D, stevioside, rebaudioside F and rebaudioside M.

[3] The production method according to [1] or [2], wherein a content of rebaudioside A is 5 to 70% by mass and a content of rebaudioside D is 2 to 70% by mass in the roughly purified product.

[4] The production method according to any of [1] to [3], wherein a concentration of the ethanol in the solvent is 99.9% by mass or less.

[5] The production method according to any of [1] to [4], wherein the solvent is kept at a temperature of 40 to 80°C in mixing of the roughly purified product.

[6] The production method according to any of [1] to [5], wherein rebaudioside D is used as a seed crystal.

[7] The production method according to any of [1] to [6], wherein the solution for crystallization is cooled to a temperature of 35°C or less with stirring, to precipitate rebaudioside D.

[8] The production method according to any of [1] to [7], wherein the solution for crystallization is cooled at a rate of 0.002 to 1.37°C/min with stirring, to precipitate rebaudioside D.

[9] The production method according to any of [1] to [8], wherein the solution for crystallization is cooled for a period of 1 to 48 hours with stirring, to precipitate rebaudioside D.

[10] The production method according to any of [1] to [9], wherein the roughly purified product is obtained by a method comprising

an extraction step of extracting a dry leaf of a stevia plant with a solvent to obtain an extract,
a solid-liquid separation step of subjecting the extract to a solid-liquid separation treatment to obtain a clarified liquid,
an flocculation step of adding a coagulant to the clarified liquid for flocculation, to obtain a treatment liquid,
a resin purification step of treating the treatment liquid with a hydrophobic porous resin, and
a concentration step of concentrating a solution after purification with the resin.

[11] The production method according to any of [1] to [10], further comprising a step of separating rebaudioside D precipitated and a liquid phase, and drying rebaudioside D separated.

[12] The production method according to any of [1] to [10], further comprising

a step of separating and drying rebaudioside D precipitated, to obtain a primary crystallized product,
a step of mixing the primary crystallized product in a solvent containing ethanol and having a methanol concentration of 1 mg/L or less, to adjust a primary crystallized product-dissolved liquid,
a step of cooling the primary crystallized product-dissolved liquid with stirring, to precipitate rebaudioside D, and
a step of separating and drying rebaudioside D precipitated.

[13] The production method according to any of [1] to [12], wherein a methanol content of a rebaudioside D-containing crystallized product is less than 10 ppm.

[14] The production method according to any of [1] to [13], wherein a ratio MeOH/EtOH of methanol to ethanol contained in a rebaudioside D-containing crystallized product is 0.00010 to 0.00080.

[15] The production method according to any of [1] to [14], wherein a ratio of rebaudioside D to the total steviol glycoside in a rebaudioside D-containing crystallized product is 35 to 95% by mass.

[16] The production method according to [15], wherein a ratio of rebaudioside A to the total steviol glycoside in the rebaudioside D-containing crystallized product is 10 to 50% by mass.

[17] The production method according to any of [1] to [16], wherein a ratio of rebaudioside D crystallized, to rebaudioside D contained in a roughly purified product, in the case of single crystallization, is 70 to 99% by mass.

[18] The production method according to any of [1] to [17], wherein, in the step of adjusting the solution for crystallization, the roughly purified product is mixed in the solvent containing ethanol such that a degree of supersaturation of rebaudioside D at 10°C is 10 or more and a degree of supersaturation of rebaudioside A at 10°C is 18 or less.

[19] A rebaudioside D-containing crystallized product, having a methanol content of less than 10 ppm.

[20] The rebaudioside D-containing crystallized product according to [19], wherein a ratio MeOH/EtOH of methanol to ethanol contained is 0.00010 to 0.00080.

[21] A rebaudioside D-containing crystallized product produced by the production method according to any of [1] to [18].

[22] A food or drink product comprising the rebaudioside D-containing crystallized product according to any of [19] to [21].

[23] The food or drink product according to [22], which is a drink.

Advantageous Effects of Invention

In the present invention, a RebD-containing crystallized product can be obtained at a high purity and a high yield ratio by use of an ethanol-based solvent.

Brief Description of Drawings

[Figure 1] Solubility curves of various concentrations in an ethanol solvent in Experimental Example 1 are illustrated.
[Figure 2] A relationship between a rate of addition of RebD and a yield ratio in Experimental Example 2 is illustrated.
[Figure 3] The effect of the concentration of an ethanol solvent on the compositional ratio of a RebD crystallized product in Experimental Example 3 is illustrated.
[Figure 4] The effect of the concentration of an ethanol solvent on the yield (amount of crystal) in Experimental Example 3 is illustrated.
[Figure 5] A temperature profile in Experimental Example 4 is illustrated.
[Figure 6] The effect of the cooling rate on the compositional ratio of a RebD crystallized product in Experimental Example 4 is illustrated.
[Figure 7] The effect of the cooling rate on the yield (amount of crystal) in Experimental Example 4 is illustrated.
[Figure 8] A temperature profile in Experimental Example 5 is illustrated.
[Figure 9] The effect of the crystallizing time on the compositional ratio of a RebD crystallized product in Experimental Example 5 is illustrated.
[Figure 10] The effect of the crystallizing time on the yield (amount of crystal) in Experimental Example 5 is illustrated.
[Figure 11] The effect of the concentration of an ethanol solvent on the compositional ratio of a RebD crystallized product in the case of secondary crystallization performed, in Experimental Example 6, is illustrated.
[Figure 12] The effect of the concentration of an ethanol solvent on the yield (amount of crystal) in the case of secondary crystallization performed, in Experimental Example 6, is illustrated.
[Figure 13] A temperature profile in secondary crystallization in Experimental Example 14 is illustrated.

Description of Embodiments

Hereinafter, the present invention will be described in detail. The following embodiments are illustrative for describing the present invention, and are not intended to limit the present invention only to these embodiments. The present invention can be carried out in various modes without departing from the gist thereof. All Literatures, Patent Application Publications, Patent Publications, and other Patent Literatures cited herein are herein incorporated by reference.
Herein, "rebaudioside", "Reb" and "Reb." represent the same meanings, and all thereof mean "rebaudioside". The same applies to "dulcoside" herein.
The present invention relates to a method for producing a rebaudioside D (RebD)-containing crystallized product by dissolving a roughly purified product obtained by roughly purifying an extract from a stevia plant (hereinafter, sometimes simply referred to as "roughly purified product".) in an ethanol-based solvent to precipitate RebD as a crystal.
A method widely performed in the art has involved first purifying RebA from an extract of a stevia plant and purifying RebD from the remaining mother liquid. The reason is because RebA is more included than RebD in a leaf of a most stevia plant available. However, in the present invention, RebD is crystallized from the stage of primary crystallization. Given that RebA is contained at a higher concentration than RebD in a roughly purified product of an extract from a stevia plant, it is surprising that a RebD-containing crystallized product with a high purity is obtained in spite of use of such a roughly purified product as a raw material.
RebA is predominantly higher in content than RebD in a leaf of a stevia plant, and thus it is considered that RebD is entrained in RebA crystallization in a conventional method. Thus, in a case where a high-purity RebD formulation has been tried to be obtained from a mother liquid after RebA crystallization, by performing RebD purification in a conventional method, RebD has been entrained in RebA crystallization and thus has been necessarily lowered in yield ratio. In the present invention, objective purification of RebD has been performed to thereby solve both the above problems and realize both a high purity and a high yield ratio of RebD.

<Rough purification>

A roughly purified product of a stevia plant is used in the production method of the present invention. The roughly purified product of a stevia plant may be a commercially available product, or may be obtained by purifying an extract from a stevia plant, in particular, a leaf of a stevia plant. The roughly purified product here used may also be one obtained by further adding RebD to such a commercially available product or one obtained by rough purification of an extract.

In a case where the roughly purified product is obtained by roughly purifying an extract from a stevia plant, the extract from a stevia plant may be a commercially available product, or may be obtained by an extraction treatment of a stevia plant, in particular, a leaf of a stevia plant. Examples of a suitable method for obtaining the roughly purified product include a method including

(A) an extraction step of extracting a dry leaf of a stevia plant with a solvent to obtain an extract,
(B) a solid-liquid separation step of subjecting the extract to a solid-liquid separation treatment to obtain a clarified liquid,
(C) an flocculation step of adding a coagulant to the clarified liquid for flocculation, to obtain a treatment liquid,
(D) a resin purification step of treating the treatment liquid with a hydrophobic porous resin, and
(E) a concentration step of concentrating a solution after the purification treatment with the resin.

Hereinafter, this method may be called the rough purification method of the present invention. Each of the steps will be described below.

In one aspect of the rough purification method of the present invention, the water content ratio of the dry leaf of a stevia plant is 1 to 11% by weight.

(A) Extraction from dry leaf of stevia plant

One aspect of the rough purification method of the present invention includes extracting a dry leaf of a stevia plant with an aqueous solvent to obtain an extract (extraction liquid). The dry leaf of a stevia plant herein refers to a fresh leaf of a stevia plant, the leaf being dried and thus decreased in water content. The water content ratio of the dry leaf of a stevia plant is preferably 1 to 10% by weight, more preferably, 2 to 8% by weight, particularly preferably 3 to 4% by weight.

The dry leaf of a stevia plant is not particularly limited as long as it includes steviol glycoside, and is preferably one in which the content of RebD or rebaudioside M (RebM) is higher than that of a dry leaf of a natural stevia plant. Such dry leaf of a stevia plant can be obtained by, for example, a method described in, for example, International Publication No. WO 2019/074089.

The preferable dry leaf of a stevia plant for use in the production method of the present invention, contains 5.0 to 25 g of steviol glycoside in 100 g of the dry leaf when the water content ratio is 3 to 4% by weight. The dry leaf in other aspects may contain 6.0 to 24 g, 7.0 to 23 g, 8.0 to 22 g, 9.0 to 21 g, 10 to 20 g, or 11 to 19 g of steviol glycoside in 100 g of the dry leaf when the water content ratio is 3 to 4% by weight.

In another preferable aspect, the dry leaf of a stevia plant, for use in the production method of the present invention, contains 8 to 17 g of steviol glycoside in 100 g of the dry leaf when the water content ratio is 3 to 4% by weight.

The preferable dry leaf in the present aspect contains 0.5 g or more of RebD in 100 g of the dry leaf when the water content ratio is 3 to 4% by weight. The dry leaf preferred for use in other aspects contains 0.19 g or more of RebM in 100 g of the dry leaf when the water content ratio is 3 to 4% by weight. The preferale dry leaf for use in other aspects contains 3.0 g or more of rebaudioside A (RebA) in 100 g of the dry leaf when the water content ratio is 3 to 4% by weight. In other preferable aspects, when the water content ratio of the dry leaf is 3 to 4% by weight, RebD is present in the dry leaf in an amount of 0.6 g or more, 0.7 g or more, 0.8 g or more, 1.0 g or more, 1.1 g or more, 1.2 g or more, 1.3 g or more, 1.4 g or more, 1.5 g or more, 1.6 g or more, 1.7 g or more, 1.8 g or more, 1.9 g or more, 2.0 g or more, 2.1 g or more, 2.2 g or more, 2.3 g or more, 2.4 g or more, 2.5 g or more, 2.6 g or more, 2.7 g or more, 2.8 g or more, 2.9 g or more, 3.0 g or more, 3.1 g or more, 3.2 g or more, 3.3 g or more, 3.4 g or more, 3.5 g or more, 3.6 g or more, 3.7 g or more, 3.8 g or more, 3.9 g or more, 4.0 g or more, 4.1 g or more, or 4.2 g or more in 100 g of the dry leaf, and may be present in an amount of, for example, 6.0 g or less, 5.5 g or less, or 5.0 g or less. Alternatively, RebD may be present in an amount of 2.7 to 8.0 g, 3.2 to 8.0 g, 3.7 to 8.0 g, 4.2 to 8.0 g, 4.7 to 8.0 g, 5.2 to 8.0 g, 5.7 to 8.0 g, 6.2 to 8.0 g, 6.7 to 8.0 g, 2.7 to 7.5 g, 3.2 to 7.5 g, 3.7 to 7.5 g, 4.2 to 7.5 g, 4.7 to 7.5 g, 5.2 to 7.5 g, 5.7 to 7.5 g, 6.2 to 7.5 g, 6.7 to 7.5 g, 2.7 to 7.0 g, 3.2 to 7.0 g, 3.7 to 7.0 g, 4.2 to 7.0 g, 4.7 to 7.0 g, 5.2 to 7.0 g, 5.7 to 7.0 g, 6.2 to 7.0 g, or 6.7 to 7.0 g. In other preferable aspects, RebM is present in the dry leaf in an amount of 0.20 g or more, 0.25 g or more, 0.30 g or more, 0.35 g or more, 0.40 g or more, 0.45 g or more, 0.50 g or more, 0.55 g or more, 0.60 g or more, 0.65 g or more, 0.70 g or more, 0.75 g or more, 0.80 g or more, 0.85 g or more, 0.90 g or more, 0.95 g or more, 1.00 g or more, 1.05 g or more, 1.10 g or more, 1.15 g or more, 1.20 g or more, 1.25 g or more, 1.30 g or more, 1.35 g or more, 1.40 g or more, or 1.45 g or more per 100 g of the dry leaf, and may be present in an amount of, for example, 1.50 g or less, 1.30 g or less, or 1.20 g or less. In other preferable aspects, when the water content ratio of the dry leaf is 3 to 4% by weight, RebA is present in an amount of 3.0 g or more, 3.5 g or more, 4.0 g or more, 4.5 g or more, 5.0 g or more, 5.5 g or more, 6.0 g or more, 6.5 g or more, 7.0 g or more, 7.5 g or more, 8.0 g or more, 8.5 g or more, 9.0 g or more, 9.5 g or more, 10 g or more, 11 g or more, 12 g or more, 13 g or more, or 14 g or more in 100 g of the dry leaf, and may be present in an amount of, for example, 17 g or less, 16 g or less or 15 g or less.

In another preferable aspect, the dry leaf may contain 0.5 to 0.9 g or 0.6 to 0.85 g of RebD in 100 g of the dry leaf when the water content ratio is 3 to 4% by weight. RebM may be contained in 100 g of the dry leaf having a water content ratio of 3 to 4% by weight, in any amount of 0.2 to 1.5 g, 0.2 to 1.3 g, 0.2 to 1.2 g, 0.2 to 1 g, 0.2 to 0.8 g, 0.2 to 0.7 g, 0.3 to 1.5 g, 0.3 to 1.3 g, 0.3 to 1.1 g, 0.3 to 0.9 g, and 0.3 to 0.7 g. RebA may be contained in 100 g of the dry leaf having a water content ratio of 3 to 4% by weight, in any amount of 3 to 17 g, 3 to 16 g, 3 to 15 g, 3 to 14 g, 3 to 13 g, 3 to 12 g, 5 to 17 g, 5 to 16 g, 5 to 15 g, 5 to 14 g, 5 to 13 g, 5 to 12 g, 6 to 17 g, 6 to 16 g, 6 to 15 g, 6 to 14 g, 6 to 13 g, 6 to 12 g, and 6 to 11 g.

Extraction of steviol glycoside from the dry leaf can be performed by use of a solvent such as water or alcohol, or a mixed solution thereof. Examples of a preferable extraction solvent include ion-exchange water, pure water (for example, milliQ water) and an aqueous ethanol solution. In extraction, the dry leaf may be pulverized. In the case of pulverization, the pulverization may be performed with a ball mill or the like. Alternatively, the extraction treatment may be performed with a kneader extractor (for example, SKN-R100, manufactured by Sanyukiki Co., Ltd.) or the like.

In the extraction, steviol glycoside can be more efficiently extracted by heating an aqueous solvent. The temperature in the extraction may be, for example, 0 to 100°C, 20 to 90°C, 40 to 80°C, 25 to 80°C, 30 to 75°C, 35 to 70°C, 40 to 65°C or 45 to 70°C, and is preferably 45 to 70°C.

The extraction may be performed not only once, but multiple times. The extraction is performed multiple times to thereby extract more steviol glycoside contained in the leaf. The extraction is preferably performed twice from the viewpoint of efficiency.

(B) Solid-liquid separation treatment

In one aspect of the rough purification method of the present invention, a clarified liquid can be obtained by subjecting the resulting extraction liquid to a solid-liquid separation treatment. The solid-liquid separation treatment is not particularly limited as long as a solid and a liquid are sufficiently separated, and examples include a treatment with a centrifuge, a treatment with a mesh, and a treatment by filter pressing.

The solid-liquid separation treatment may be performed by using a plurality of units, and a clarified liquid may be obtained by, for example, performing a first solid-liquid separation treatment and then a second solid-liquid separation treatment.

Examples of other solid-liquid separation treatment than the above-mentioned treatments can also include a treatment with a microfiltration membrane.

(C) Flocculation treatment

In one aspect of the rough purification method of the present invention, a treatment liquid can be obtained by adding a coagulant to the clarified liquid obtained by the solid-liquid separation treatment. The coagulant is not particularly limited, and a known inorganic coagulant or organic polymeric coagulant can be used. In other aspects of the present invention, the coagulant corresponds to one or more selected from aluminum sulfate, polyaluminum chloride, iron chloride (III) or a hydrate thereof, a synthetic polymeric coagulant (a polyacrylamide high polymer, a partially hydrolyzed product of polyacrylamide, or the like), alginic acid, chitin, chitosan, and calcium hydroxide. One or more of such coagulants may be included in the coagulant, or other coagulant may also be further included therein. The coagulant may be used in combinations of two or more kinds thereof, and, for example, a combination of calcium hydroxide and iron chloride, or a combination of calcium hydroxide, iron chloride and chitosan may also be used.

The amount of the coagulant added is not particularly limited as long as the coagulant is flocculated, and the coagulant can be added in an amount of, for example, 3.0 to 50% by weight based on a soluble solid content included in the clarified liquid. For example, calcium hydroxide can be added in an amount corresponding to 10 to 30% by weight based on the solid content in the clarified liquid, and can be preferably added in an amount of 12 to 28% by weight, more preferably 14 to 25% by weight. Iron chloride (III) hexahydrate can be added in an amount corresponding to 15 to 40% by weight based on the solid content in the clarified liquid, and can be preferably added in an amount of 18 to 38% by weight, more preferably 20 to 35% by weight. In the case of a 0.5% (w/v) chitosan solution, the solution can be added in an amount corresponding to 3.0 to 10% by weight based on the solid content in the clarified liquid, and can be preferably added in an amount of 4.0 to 8.0% by weight, more preferably 4.5 to 7.0% by weight.

The pH in the flocculation treatment is not particularly limited, and can be appropriately selected so that flocculation is optimized depending on the type of the coagulant. In one aspect of the present invention, the pH of the clarified liquid in the flocculation treatment may be 2.0 to 13, 3.0 to 13, 4.0 to 13, 5.0 to 13, or 6.0 to 13.

The temperature of the flocculation treatment is not particularly limited, and the flocculation treatment may be performed at room temperature (about 25°C) with neither heating nor cooling.

An flocculated product included in the treatment liquid may also be removed after the flocculation treatment and before a purification treatment with a resin, described below. The flocculated product can be removed by any method such as filtration.

Examples of the method for removing the flocculated product can also include centrifugation, in addition to the above-mentioned method.

(D) Purification treatment with resin

In one aspect of the rough purification method of the present invention, the treatment liquid obtained by the flocculation treatment is treated with a hydrophobic porous resin. Steviol glycoside has a molecular structure having a hydrophilic group and a hydrophobic group and is amphiphilic, and has a molecular weight of around 1,000. Steviol glycoside is also known to be stable at a pH of 2.5 to 9.0 and not to be ionized even under acidity or basicity. On the other hand, a large amount of any component other than steviol glycoside is included in the treatment liquid obtained after the flocculation treatment. Although not bound to any theory, such any components include a component like an iron ion having a molecular weight different from that of steviol glycoside, and an ionizable component like amino acids, and it is considered that these components can be removed by a treatment with a hydrophobic porous resin.

Steviol glycoside having a hydrophobic steviol backbone is bound hydrophobically to and trapped by a synthetic resin. In contrast, impurities with a high hydrophilicity are not bound to the resin and are moved to a through fraction and removed, and it is thus considered that steviol glycoside has an enhanced purity by loading the treatment liquid obtained after the flocculation treatment to a column packed with the above resin, and thereafter performing washing with water. Since a bond between steviol glycoside and a functional group of the synthetic resin is dissociated by a low polar solvent, an advantage is that steviol glycoside can be recovered finally at a high yield ratio.

The porous resin for use in one aspect of the rough purification method of the present invention is not particularly limited as long as it is a porous resin low in affinity with water, and is preferably, for example, a porous resin of one or more hydrophobic resins selected from a copolymer of styrene and divinylbenzene, polyethylene, polypropylene, polystyrene, poly(meth)acrylonitrile, polyamide, and polycarbonate. In a preferable aspect of the present invention, a copolymer of styrene and divinylbenzene is preferably subjected to no introduction treatment of any ion exchange group (namely, has no ion exchange group). In general, while, when an ion exchange resin is produced, styrene and divinylbenzene are copolymerized to form a cubic network structure and then an ion exchange group is introduced into the resin, the "subjected to no introduction treatment of any ion exchange group" means that such a treatment is not conducted.

In one aspect of the rough purification method of the present invention, the hydrophobic porous resin has a hydrophobic group, and the hydrophobic group contains one or more selected from an aryl group, an alkyl group, an alkylsilyl group, an ester group and an epoxy group. As long as one or more hydrophobic groups selected therefrom are contained, any other hydrophobic group may also be further contained. Examples of the aryl group include a phenyl group, a benzyl group, a tolyl group, and a xylyl group, and examples of the alkyl group include C1 to 20 alkyl groups such as a methyl group, an ethyl group, a propyl group, a butyl group, a pentyl group, and an octadecyl group.

In a preferable aspect of the rough purification method of the present invention, the most frequent pore radius of the hydrophobic porous resin is 10 to 200 Å. In a preferable aspect, the most frequent pore radius is 10 to 150 Å, 15 to 100 Å, or 20 to 80 Å. It is considered that such pore characteristics are exhibited to thereby allow steviol glycoside to efficiently adsorb to pores and be efficiently separated from other components.

The treatment liquid may also be treated with an anion exchange resin before the treatment with the hydrophobic porous resin is performed. Such a treatment with an anion exchange resin can be performed in advance to thereby effectively remove a component bound to the hydrophobic resin, such as a dye and/or catechin. Such an anion exchange resin is not particularly limited, examples thereof include a basic anion exchange resin, and such a basic anion exchange resin here used can be, for example, a weakly basic anion exchange resin into which a primary to secondary amino group is introduced as a functional group, or a strongly basic anion exchange resin having a quaternary ammonium group (for example, a trimethylammonium group or a dimethylethanol ammonium group) .

(E) Concentration treatment

The solution obtained after the purification treatment with the resin may be further subjected to a concentration treatment for removal of an aqueous solvent. Such a treatment is not particularly limited, and examples thereof include a method for evaporating the aqueous solvent by heating, or a method for removing the aqueous solvent by drying under reduced pressure.

After the concentration treatment, spray drying may also be performed. In usual temperature conditions of the spray drying, the inlet temperature is about 120 to 200°C and the outlet temperature is about 80 to 120°C.

(Roughly purified product)

The roughly purified product for use in the production method of the present invention includes the total steviol glycoside in an amount of 50 to 95% by mass based on the total weight of the roughly purified product, and suitably includes the total steviol glycoside in any amount (% by mass) of 55 to 95, 60 to 95, 70 to 95, 80 to 95, 90 to 95, 50 to 90, 50 to 80, 50 to 70, 50 to 65, 50 to 60, 55 to 90, 55 to 80, 55 to 70, 55 to 60, 60 to 70, 60 to 80, 50 to 94, 55 to 94, and 60 to 94.

In the present Description, the total steviol glycoside suitably includes seven kinds of steviol glycosides including RebA, RebB, RebC, RebD, stevioside, RebF and RebM. In the present Description, the total steviol glycoside may be abbreviated as "TSG".

The roughly purified product includes at least RebA and RebD. The content of RebA (% by mass) is suitably any of 5 to 70, 15 to 70, 20 to 70, 25 to 70, 35 to 70, 45 to 70, 55 to 70, 65 to 70, 5 to 65, 5 to 45, 5 to 25, 15 to 65, 25 to 45, 40 to 75, and 45 to 65, and the content of RebD (% by mass) is any of 1 to 70, 2 to 70, 5 to 70, 15 to 70, 25 to 70, 35 to 70, 45 to 70, 55 to 70, 65 to 70, 1 to 65, 1 to 45, 1 to 25, 2 to 65, 2 to 45, 2 to 25, 5 to 65, 5 to 45, 5 to 25, 15 to 65, 25 to 45, 40 to 75, 45 to 65, 1 to 20, 1 to 15, 1 to 10, 1 to 8, 2 to 20, 2 to 15, 2 to 10, and 2 to 8, in the roughly purified product.

In another preferable aspect, the content of RebA is 1 to 30% by mass and the content of RebD is any of the above, in the roughly purified product.

<Preparation of solution for crystallization>

Subsequently, a solvent is placed in any container, for example, a crystal can provided with a stirring blade, and the roughly purified product is mixed in the solvent, to prepare a solution for crystallization. The degree of supersaturation of RebA and the degree of supersaturation of RebD are here set so that no RebA is formed into a crystal. The roughly purified product is then mixed in the solvent in an amount so that such degrees of supersaturation are achieved.

The degree of supersaturation is represented by the following formula.

$$\sigma = (C - C^*)/C^*$$

In the formula, $\sigma$ represents the degree of supersaturation, C represents the ratio of the amount of a substance added to the solvent (ppm), and $C^*$ represents the saturated dissolution concentration (ppm).

The reason why C herein represents not simply "concentration of a substance", but "ratio of the amount of a substance added to the solvent" is because a case is expected where a substance added is not completely dissolved and is in the state of being suspended. The ratio of the amount added to the solvent, is herein adopted as a standard of the degree of supersaturation regardless of the presence or absence of dissolution of a substance added and the degree thereof.

A seed crystal may be used in the present invention, but the amount of the seed crystal added is not involved in calculation of the degree of supersaturation.

The saturated solubility is herein measured according to the following method. That is, 100 ml of a solvent having a predetermined ethanol concentration (w/w) is placed in a container (having a volume of 100 ml or 200 ml, suitably 200 ml), the liquid temperature is set to 10°C by a water bath in advance, and then the solvent is stirred at 200 rpm, and a target substance is loaded in an excessive amount (amount until suspension is made). After 24 hours, a filtrate is obtained by a 0.45-um membrane filter, and the amount of the target substance (RebA or RebD) included in the filtrate is measured with LCMS. LCMS here used is suitably LCMS 8050 manufactured by Shimadzu Corporation.

The degree of supersaturation of RebD (10°C) is preferably 10 or more, and is more suitably any of 10 to 200, 10 to 180, 10 to 160, 10 to 140, 10 to 120, 10 to 100, 10 to 80, 10 to 60, 10 to 40, 10 to 20, 15 to 200, 35 to 200, 55 to 200, 75 to 200, 95 to 200, 115 to 200, 135 to 200, 155 to 200, 175 to 200, 15 to 180, 15 to 160, 15 to 140, 15 to 120, 15 to 100, 15 to 80, 15 to 60, 15 to 40, 15 to 20, 20 to 180, 20 to 160, 20 to 140, 20 to 120, 20 to 100, 20 to 80, 20 to 60, 20 to 40, 80 to 180, 80 to 160, 80 to 140, 80 to 120, 80 to 100, 100 to 180, 100 to 160, 100 to 140, and 100 to 120.

The degree of supersaturation (10°C) means the degree of supersaturation in the case of 10°C as a standard temperature. It is not meant that cooling at 10°C is necessary.

In another preferable aspect, the degree of supersaturation of RebD (10°C) is 10 to 350, 20 to 350, 50 to 350, 10 to 300, 20 to 300, 50 to 300, 10 to 205, or 20 to 205.

The degree of supersaturation of RebA (10°C) is 18 or less and preferably also includes 0 or less, and the degree of supersaturation of RebA (10°C) is more preferably more than 0 and 18 or less, and particularly suitably any of 3 to 18, 3 to 13, 3 to 8, 5 to 18, 7 to 18, 9 to 18, 11 to 18, 13 to 18, 15 to 18, 17 to 18, 5 to 13, 7 to 11, 5 to 15, 7 to 15, 9 to 15, 11 to 15, and 13 to 15.

In another preferable aspect, the degree of supersaturation of RebA (10°C) is any of 16 or less, -1 to 16, and 0 to 16.

RebA is hardly precipitated and RebD is easily precipitated in a condition where the degree of supersaturation of RebA is low and the degree of supersaturation of RebD is high. In a case where both the degrees of supersaturation of RebA and RebD (10°C) are within the above ranges and the degree of supersaturation of RebA is more than 0, the ratio $\sigma D/\sigma A$ of the degree $\sigma D$ of supersaturation of RebD to the degree $\sigma A$ of supersaturation of RebA is preferably any of 4 or more, 5 or more, 4 to 40, 5 to 40, 7 to 40, 12 to 40, 17 to 40, 22 to 40, 27 to 40, 32 to 40, 37 to 40, 4 to 30, 5 to 30, 7 to 30, 12 to 30, 17 to 30, 22 to 30, 27 to 30, 4 to 35, 5 to 35, 8 to 35, 13 to 35, 18 to 35, 23 to 35, 28 to 35, 33 to 35, 8 to 25, 13 to 25, 18 to 25, 23 to 25, 10 to 40, 15 to 40, 20 to 40, 25 to 40, 30 to 40, 35 to 40, 10 to 30, 15 to 30, 20 to 30, and 25 to 30.

In another preferable aspect, the $\sigma D/\sigma A$ is any of 4 to 1,000,000, 5 to 1,000,000, 4 to 750,000, and 5 to 750,000.

The solvent for use in adjustment of the solution for crystallization is an ethanol solvent. An aqueous ethanol solution is suitable. The ethanol solvent for use in the present invention is substantially free of methanol, and the methanol concentration of the solvent is specifically 1 mg/L or less, more suitably 0.5 mg/L or less, particularly suitably 0.1 mg/L or less. The lower limit of the methanol concentration is 0 mg/L. Ethanol is more environment-friendly and additionally lower in effect on the human body, than methanol. While use of methanol imposes ventilation installation and ventilation capacity in a manufacturing setting, and implementation of the special medical examination for employees, by law, use of ethanol has a large advantage in terms of an industrial application because of not imposing them.

The amount of the roughly purified product added is appropriately set so that the degree of supersaturation falls within the above-mentioned range, and the roughly purified product is suitably mixed in an amount of 1 to 30% by mass, more suitably an amount corresponding to any amount (% by mass) per solvent of 1 to 25, 1 to 20, 1 to 15,1 to 10, 5 to 30, 5 to 25, 5 to 20, 5 to 15, 5 to 10, 10 to 30, 15 to 30, 20 to 30, 25 to 29, 10 to 25, and 15 to 20.

In another preferable aspect, the amount of the roughly purified product added, per solvent, is 1 to 6% by mass, 6 to 25% by mass, 6 to 28% by mass or 1 to 28% by mass.

The amount of the roughly purified product added is appropriately set so that the degree of supersaturation falls within the above-mentioned range, and the roughly purified product is suitably added in an amount so that the amount of RebD per solvent is any amount (% by mass) of 0.15 to 3.50, 0.30 to 3.50, 0.45 to 3.50, 0.60 to 3.50, 0.75 to 3.50, 0.90 to 3.50, 1.05 to 3.50, 1.20 to 3.50, 1.50 to 3.50, 1.80 to 3.50, 2.10 to 3.50, 2.40 to 3.50, 2.70 to 3.50, 3.00 to 3.50, 0.15 to 3.00, 0.30 to 3.00, 0.45 to 3.00, 0.60 to 3.00, 0.75 to 3.00, 0.90 to 3.00, 1.05 to 3.00, 1.20 to 3.00, 1.50 to 3.00, 1.80 to 3.00, 2.10 to 3.00, 2.40 to 3.00, 2.70 to 3.00, 0.15 to 2.50, 0.15 to 2.00, 0.15 to 1.50, 0.15 to 1.00, 0.15 to 0.70, 0.15 to 0.40, 0.30 to 2.50, 0.45 to 2.00, 0.60 to 1.50, and 0.75 to 1.00.

In another preferable aspect, the roughly purified product is added in an amount so that the amount of RebD per solvent is any amount (% by mass) of 0.20 to 3.50 and 0.25 to 3.50.

The concentration of the ethanol solvent (% by mass) is suitably 99.9% by mass or less, more suitably 92% by mass or less. In one aspect of the present invention, the concentration is any of 50 to 99, 60 to 99, 70 to 99, 80 to 99, 90 to 99, 50 to 89, 60 to 79, 50 to 92, 60 to 92, 70 to 92, 80 to 92, 90 to 92, 50 to 82, 60 to 72, 70 to 90, 80 to 90, and 70 to 80% by mass.

In another preferable aspect, the concentration of a solvent containing methanol or ethanol (% by mass) is any of 73% by mass or more and less than 95% by mass, and 73 to 92% by mass.

When the roughly purified product is mixed in the solvent, the liquid temperature (°C) of the solvent is preferably kept at any of 40 to 80, 42 to 80, 44 to 80, 46 to 80, 48 to 80, 50 to 80, 52 to 80, 57 to 80, 62 to 80, 67 to 80, 72 to 80, 40 to 75, 40 to 70, 40 to 65, 40 to 60, 40 to 55, 40 to 50, 42 to 75, 44 to 70, 46 to 65, 48 to 60, and 50 to 55.

When the roughly purified product is mixed in the solvent, suitably, a seed crystal is also preferably mixed. The seed crystal here used is RebD. The size of the seed crystal is not particularly limited.

In a case where the seed crystal is added, the solution temperature is preferably one leading to supersaturation from the viewpoint of avoidance of dissolution of the seed crystal.

The amount of the seed crystal added may be appropriately determined depending on other conditions and the like, and a larger amount added tends to lead to a more enhancement in yield ratio. Suitably, the rate of addition of the seed crystal Cs, represented by the following formula, is preferably any value of 0.050 to 10.000, 0.075 to 10.000, 0.100 to 10.000, 0.200 to 10.000, 0.300 to 10.000, 0.400 to 10.000, 0.500 to 10.000, 0.600 to 10.000, 0.700 to 10.000, 0.800 to 10.000, 0.900 to 10.000, 1.000 to 10.000, 2.000 to 10.000, 3.000 to 10.000, 4.000 to 10.000, 5.000 to 10.000, 6.000 to 10.000, 7.000 to 10.000, 8.000 to 10.000, 0.050 to 9.000, 0.050 to 8.000, 0.050 to 7.000, 0.050 to 6.000, 0.050 to 5.000, 0.050 to 4.000, 0.050 to 3.000, 0.050 to 2.000, 0.050 to 1.000, 0.050 to 0.700, 0.050 to 0.400, 0.050 to 0.100, 0.075 to 9.000, 0.100 to 8.000, 0.200 to 7.000, 0.300 to 6.000, 0.400 to 5.000, 0.500 to 4.000, 0.600 to 3.000, 0.700 to 2.000, 0.800 to 1.000, 5.500 to 9.500, 6.500 to 9.500, 7.500 to 9.500, 8.500 to 9.500, 5.500 to 8.500, and 6.500 to 7.500.

$$Cs = Ws/W_{th}$$

In the formula, Ws represents the amount of the seed crystal (unit: g), and $W_{th}$ represents the yield expected (unit: g).

$$W_{th} = (C - C^*)/1,000,000 \times \text{Amount of solvent}$$

In the formula, C represents the ratio of the amount of RebD added to the solvent (ppm), and C* represents the saturated solubility (unit: ppm).
The unit of the amount of the solvent is ml.
C does not include the amount of the seed crystal.

<Precipitation>

RebD is precipitated by cooling the solution for crystallization with stirring in any crystallization apparatus, for example, a crystallizer such as a cylindrical rotary crystallizer.
Cooling, if rapidly performed, can cause a crystallized product with a low purity of RebD to be obtained or can lead to no enhancement in yield ratio, and thus cooling is preferably gradually performed. The cooling rate (°C/min) is suitably 0.002°C/min or more, and is more suitably 0.002 to 1.37°C/min, in particular, any of 0.002 to 1.37, 0.002 to 0.87, 0.002 to 0.37, 0.002 to 0.27, 0.002 to 0.17, 0.002 to 0.07, 0.002 to 0.04, 0.002 to 0.01, 0.002 to 0.007, 0.007 to 1.37, 0.017 to 1.37, 0.027 to 1.37, 0.057 to 1.37, 0.087 to 1.37, 0.387 to 1.37, 0.687 to 1.37, 0.987 to 1.37, 0.007 to 0.87, 0.017 to 0.37, 0.027 to 0.27, and 0.057 to 0.17.
In another preferable aspect, the cooling rate (°C/min) is 0.02 to 0.2.
The stirring and cooling time (unit: hour) may be appropriately set depending on other conditions, for example, whether or not the seed crystal is used, and is preferably any of 1 to 48, 6 to 48, 11 to 48, 16 to 48, 21 to 48, 26 to 48, 31 to 48, 36 to 48, 41 to 48, 46 to 48, 1 to 43, 1 to 38, 1 to 33, 1 to 28, 1 to 23, 1 to 18, 1 to 13, 1 to 8, 1 to 3, 6 to 43, 11 to 38, 16 to 33, and 21 to 28, from the viewpoint of an enhancement in yield ratio.
In another preferable aspect, the stirring and cooling time (unit: hour) is 6 to 24 hours, 12 hours or more, or 12 to 24 hours.
The cooling temperature (°C) in the precipitation step is appropriately determined depending on other conditions, for example, whether or not the seed crystal is used, and cooling to any of 3 to 40, 6 to 40, 9 to 40, 12 to 40, 15 to 40, 18 to 40, 21 to 40, 24 to 40, 27 to 40, 30 to 40, 33 to 40, 20 to 40, 35°C or less, 3 to 35, 6 to 35, 9 to 35, 12 to 35, 15 to 35, 18 to 35, 21 to 35, 24 to 35, 27 to 35, 30 to 35, and 25 to 35 is preferable.
In another preferable aspect, cooling is performed to a cooling temperature (°C) of 9 to 20°C, 9 to 17°C, 4 to 20°C or 4 to 17°C.
The temperature (°C) at the start of cooling is preferably any of 30 to 85, 40 to 85, 50 to 85, 60 to 85, 70 to 85, 30 to 80, 30 to 70, 30 to 60, 30 to 50, 30 to 40, 40 to 80, 40 to 70, and 40 to 60.
The stirring speed (rpm) is preferably any of 5 to 600, 5 to 500, 5 to 400,5 to 300,5 to 200,5 to 100,5 to 50,5 to 20,55 to 600,105 to 600,155 to 600,205 to 600,255 to 600,305 to 600,355 to 600,405 to 600,455 to 600,505 to 600,555 to 600,50 to 600,200 to 600,350 to 600,500 to 600,100 to 450,100 to 300,100 to 150, 200 to 500, and 300 to 400.

<Post step>

A solid composition including RebD precipitated is separated from a liquid phase by centrifugation, filtration, or the like, and RebD after separation is dried. A separation unit is not particularly limited as long as a solid and a liquid are sufficiently separated, and examples include a treatment with a centrifuge, a treatment with a membrane filter, and a treatment with a mesh. A surface may be, if necessary, washed by spraying a RebD crystal with a small amount of the same solvent as that used in crystallization or the like, after separation and before drying.

<Other>

A mother liquid after the solid-liquid separation treatment may be utilized in crystallization of other components such as RebA and RebM.
In one aspect of the production method of the present invention, the above-mentioned rough purification step, preparation step of a solution for crystallization, precipitation step and post step may be each performed once for completion of production (single crystallization), from the viewpoint that the yield ratio is more emphasized than the purity. In other aspects, steps from the preparation step of a solution for crystallization to the post step may be repeated multiple times (multiple crystallization) in order to more enhance the purity of a RebD-containing crystallized product. Double crystallization is preferable from the viewpoint of the balance between the yield ratio and the purity.

<Double crystallization>

In a case where double crystallization is performed, a RebD-containing crystallized product can be obtained by performing steps from the rough purification step to the precipitation step to separate and dry rebaudioside D precipitated, to obtain a primary crystallized product, thereafter mixing the primary crystallized product, instead of the roughly purified product, in an ethanol solvent to prepare a primary crystallized product-dissolved liquid, cooling the primary crystallized product-dissolved liquid, instead of the solution for crystallization, with stirring, to precipitate RebD, and separating and drying RebD precipitated. The procedure and conditions in the double crystallization may be the same as the above-mentioned crystallization procedure and crystallization conditions. In this case, the "roughly purified product" in the above description is read as the "primary crystallized product".

In the double crystallization, the concentration of the ethanol solvent for use in preparation of the solution for crystallization can be low as compared with the case of the single crystallization, to result in a more enhancement in purity. The degree of supersaturation of RebA in second crystallization may be set lower than that in first crystallization.

In the present invention, crystallization may be performed any times, but purification at a sufficiently high purity can be made even by performing crystallization two or less times. Thus, there are various advantages of a decrease in reduction, shortening in cycle time, energy saving, and the like.


<RebD-containing crystallized product>

A RebD-containing crystallized product (hereinafter, sometimes called "RebD-containing crystallized product of the present invention".) obtained according to the method of the present invention contains a large amount of RebD. The ratio of RebD to TSG in the RebD-containing crystallized product of the present invention (% by mass) is suitably any of 35 to 99, 45 to 99, 55 to 99, 65 to 99, 75 to 99, 85 to 99, 35 to 89, 35 to 79, 35 to 69, 35 to 59, 35 to 95, 45 to 95, 55 to 95, 65 to 95, 75 to 95, 85 to 95, 40 to 99, 50 to 99, 60 to 99, 70 to 99, 80 to 99, 90 to 99, 40 to 99, 40 to 89, 40 to 79, 40 to 69, 40 to 59, 50 to 89, 60 to 79, 40 to 95, 50 to 95, 60 to 95, 70 to 95, 80 to 95, 90 to 95, 40 to 85, 40 to 75, 40 to 65, 40 to 55, 50 to 85, and 60 to 75.

The content of RebA in the RebD-containing crystallized product of the present invention is low. The ratio of RebA to TSG (% by mass) is suitably any of 5 to 80, 5 to 50, 10 to 50, 20 to 50, 30 to 50, 40 to 50, 10 to 40, 20 to 40, 20 to 30, 30 to 40, 10 to 30, 10 to 20, 12 to 18, 5 to 30, 5 to 20, and 5 to 10 in a condition where the ratio of RebD falls within any of the above numerical ranges.

In another preferable aspect, the ratio of RebA to TSG (% by mass) is 3.5 to 80, 3.5 to 35, 3.5 to 30 or 3.5 to 12.

In the method of the present invention, RebD can be crystallized at a high yield ratio. The ratio of RebD crystallized to RebD included in the roughly purified product, in the case of the single crystallization (% by mass) is suitably any of 70 to 99, 75 to 99, 80 to 99, 85 to 99, 90 to 99, 70 to 94, 70 to 89, 70 to 84, 70 to 79, 70 to 74, 75 to 94, 80 to 89, 50 to 99, 55 to 99, 60 to 99, 65 to 99, 50 to 94, 50 to 89, 50 to 84, 50 to 79, 50 to 74, 50 to 69, 55 to 94, 60 to 89, and 65 to 84.

In another preferable aspect, the ratio of RebD crystallized to RebD included in the roughly purified product, in the case of the single crystallization (% by mass) is 39 to 85, more suitably 39 to 82.

The recovery ratio (sometimes called "total yield ratio".) of RebD until completion of crystallization from extraction of a leaf is also high, and is suitably 35 to 90, 45 to 90, 55 to 90, 65 to 90, or 75 to 90% by mass.

The proportion (% by mass) of TSG in the RebD-containing crystallized product of the present invention is suitably 50 to 99, 60 to 99, 70 to 99, 80 to 99, 90 to 99, 50 to 90, 50 to 70, 50 to 60, 60 to 80, 60 to 90, 60 to 70, or 70 to 80.

In another aspect, the proportion of TSG in the RebD-containing crystallized product of the present invention (% by mass) is suitably 50 or more, 60 or more, 70 or more or 75 or more.

The amount of methanol included in the RebD-containing crystallized product of the present invention is small, and is suitably less than 10 ppm, particularly suitably less than 5 ppm, most suitably 2 ppm or less. The methanol content can be measured with head space GCMS. The lower limit value is preferably 1 ppm.

In another preferable aspect, the lower limit value of the amount of methanol included in the RebD-containing crystallized product is 0 ppm.

The ratio MeOH/EtOH of methanol to ethanol contained in the RebD-containing crystallized product of the present invention is also a low value. The ratio is suitably 0.00010 to 0.00080, more suitably 0.00010 to 0.00070, particularly suitably 0.00010 to 0.00050, most suitably 0.00010 to 0.00030.

According to one aspect of the present invention, the RebD-containing crystallized product of the present invention may be used as a sweetener composition. Such a sweetener composition may include, in addition to the RebD-containing crystallized product of the present invention, a sweetener other than steviol glycoside. Examples of such other sweetener include natural sweeteners such as fructose, sugar, fructose glucose liquid sugar, glucose, maltose, high-fructose liquid sugar, sugar alcohol, oligosaccharide, honey, sugar cane juice (brown sugar honeydew), starch syrup, a Momordica grosvenori powder, a Momordica grosvenori extract, a licorice powder, a licorice extract, a Thaumatococcus daniellii seed powder, a Thaumatococcus daniellii seed extract, and sucrose, and artificial sweeteners such as acesulfame potassium, sucralose, neotame, aspartame, and saccharin. In particular, a natural sweetener is preferably used, and in particular, fructose, glucose, maltose, sucrose, or sugar is suitably used from the viewpoint of imparting refreshing, ease of drinking, a natural flavor, and a proper rich taste. Such a sweet component may be used singly or in combinations of a plurality of kinds thereof.

One aspect of the present invention provides a food or drink product, a perfume material and a pharmaceutical product including the RebD-containing crystallized product of the present invention (herein respectively also referred to as "the food or drink product of the present invention", "the perfume material of the present invention" and "the pharmaceutical product of the present invention"). The food or drink product, the perfume material and the pharmaceutical product of the present invention are not particularly limited as long as each thereof includes the RebD-containing crystallized product of the present invention. The food or drink product here means a drink and a food, and the food or drink product means a drink in a preferable correspondence. Accordingly, in a certain embodiment, the present invention provides a novel drink or food.

The amounts of the total steviol glycoside included in the food or drink product, the perfume material and the pharmaceutical product of the present invention (ppm by mass) differ depending on a specific food or drink product, and, in the case of a drink, the amount is preferably approximately 1 to 800 ppm by mass, and may be, for example, 20 to 750, 20 to 700, 20 to 650, 20 to 600, 20 to 550, 25 to 550, 30 to 550, 35 to 550, 40 to 550, 45 to 550, 50 to 550, 55 to 550, 20 to 540, 25 to 540, 30 to 540, 35 to 540, 40 to 540, 45 to 540, 50 to 540, 55 to 540, 20 to 530, 25 to 530, 30 to 530, 35 to 530, 40 to 530, 45 to 530, 50 to 530, 55 to 530, 20 to 520, 25 to 520, 30 to 520, 35 to 520, 40 to 520, 45 to 520, 50 to 520, 55 to 520, 20 to 510, 25 to 510, 30 to 510, 35 to 510, 40 to 510, 45 to 510, 50 to 510, 55 to 510, 20 to 505, 25 to 505, 30 to 505, 35 to 505, 40 to 505, 45 to 505, 50 to 505, 55 to 505, 20 to 500, 25 to 500, 30 to 500, 35 to 500, 40 to 500, 45 to 500, 50 to 500, 55 to 500, 20 to 495, 25 to 495, 30 to 495, 35 to 495, 40 to 495, 45 to 495, 50 to 495, 55 to 495, 20 to 490, 25 to 490, 30 to 490, 35 to 490, 40 to 490, 45 to 490, 50 to 490, 55 to 490, 100 to 400, 150 to 400, 200 to 400, 250 to 400, 300 to 400, 100 to 150, 100 to 200, 100 to 250, or 100 to 300. The content set within such a range has the advantage of enabling proper sweetness to be imparted. Herein, "ppm" means "ppm by mass", unless particularly noted.

The food or drink product, the perfume material and the pharmaceutical product of the present invention may each further include a sweetener other than steviol glycoside. Examples of such other sweetener include natural sweeteners such as fructose, sugar, fructose glucose liquid sugar, glucose, maltose, sucrose, high-fructose liquid sugar, sugar alcohol, oligosaccharide, honey, sugar cane juice (brown sugar honeydew), starch syrup, a Momordica grosvenori powder, a Momordica grosvenori extract, a licorice powder, a licorice extract, a Thaumatococcus daniellii seed powder, and a Thaumatococcus daniellii seed extract, and artificial sweeteners such as acesulfame potassium, sucralose, neotame, aspartame, and saccharin. In particular, a natural sweetener is preferably used, and in particular, fructose, glucose, maltose, sucrose, or sugar is suitably used from the viewpoint of imparting refreshing, ease of drinking, a natural flavor, and a proper rich taste. Such a sweet component may be used singly or in combinations of a plurality of kinds thereof.

Examples of the food of the present invention include, but not particularly limited, confectionery, bread and buns, flour, noodles, rice foods, agricultural/forest processed foods, livestock processed foods, fishery processed foods, milk, milk products, fat and oil/fat and oil processed foods, seasoning, and other raw materials of foods.

Examples of the drink of the present invention include, but not particularly limited, carbonated drinks, non-carbonated drinks, alcohol drinks, non-alcoholic drinks, beer taste drinks such as beer and non-alcoholic beer, coffee drinks, tea drinks, cocoa drinks, energy drinks, and functional drinks.

Examples

Hereinafter, the present invention will be further specifically described with reference to Experimental Examples.

In the following Experimental Examples, TSG means seven kinds including RebA, RebB, RebC, RebD, Stevioside, RebF and RebM, unless particularly noted.

A small-scale experiment was first performed in a laboratory in order to search optimal RebD crystallization conditions, and a relationship between various experimental conditions and results was studied. The small-scale experiment was to find a general relationship between conditions and results.

<Experimental Example 1>

The degree of supersaturation has a very large effect on phenomena of nucleus generation and crystal growth. The solubilities of RebA as a main component of a stevia leaf and RebD as a target component, in a solvent, were measured.

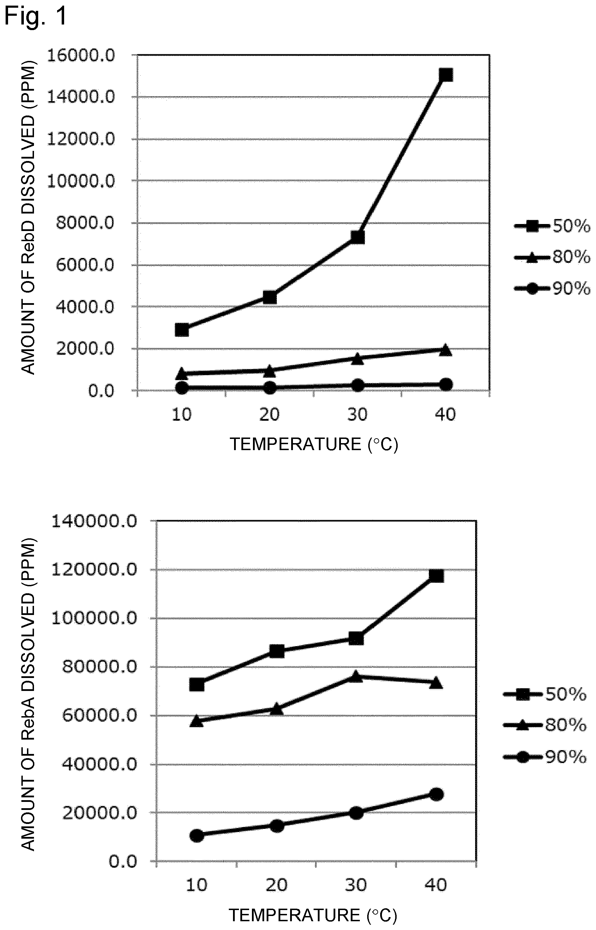

Specifically, 100 ml of a solvent having a predetermined ethanol concentration (w/w) was placed in a 50-ml or 100-ml glass container (separable round-bottomed flask, Tokyo Rikakikai Co., Ltd.), set to a predetermined temperature by a water bath (PCC-7000, Tokyo Rikakikai Co., Ltd.) in advance, and then stirred (SPZ-100, Tokyo Rikakikai Co., Ltd.) at 200 rpm, and RebA (J-100, Morita Kagaku Kogyo Co., Ltd.) or RebD (Jining Renewal & Joint International, China) was loaded in an excessive amount (amount until suspension was achieved). After 24 hours, a filtrate was obtained by a 0.45-um membrane filter, the amount of RebA or RebD included in the filtrate was measured with LCMS (LCMS 8050 manufactured by Shimadzu Corporation), and the solubility curve was created. The results are illustrated in Figure 1. According to Figure 1, the respective saturated dissolution concentrations of RebA and RebD in a 90% ethanol solvent at 10°C were 8,250 ppm and 99.3 ppm.

<Experimental Example 2>

In the present experiment, the effect of the ratio of RebD to a solvent on the recovery ratio of RebD was confirmed.
An experiment in a one component system of RebD was performed. Specifically, unmodified alcohol (99.9% by mass grade) was used, water was appropriately added thereto to adjust the ethanol concentration, and thus a 50% by mass ethanol solvent was prepared. A separable round-bottomed flask (manufactured by Tokyo Rikakikai Co., Ltd.) was used to dissolve RebD in a 50% by mass ethanol solvent under conditions in the following Table. RebD was here added as a seed crystal in an amount so that the rate of addition of the seed crystal relative to the yield expected was 0.05. Next, cooling was made under the following conditions.

(Crystallization conditions)

Initial Temp 45°C
End Temp 10°C
RebD manufactured by Jining Renewal & Joint International
Agitation speed 100 rpm

[Table 1]

| Sample RebD (g) | Solvent (ml) | Rate of addition of seed (Cs) | Seed (g) |
|---|---|---|---|
| 1. 47 | 100 | 0.05 | 0. 0665 |
| 1. 17 | 100 | 0.05 | 0. 0515 |
| 0. 96 | 100 | 0.05 | 0. 0353 |

The rate of addition of a seed crystal is calculated according to the following formula.

$$C_s = W_s/W_{th}$$

In the formula, $W_s$ represents the amount of the seed crystal (unit: g), $W_{th}$ represents the yield expected (g), and $C_s$ represents the rate of addition of the seed crystal (no unit).

$$W_{th} = (C-C^*)/1,000,000 \times \text{Amount of solvent}$$

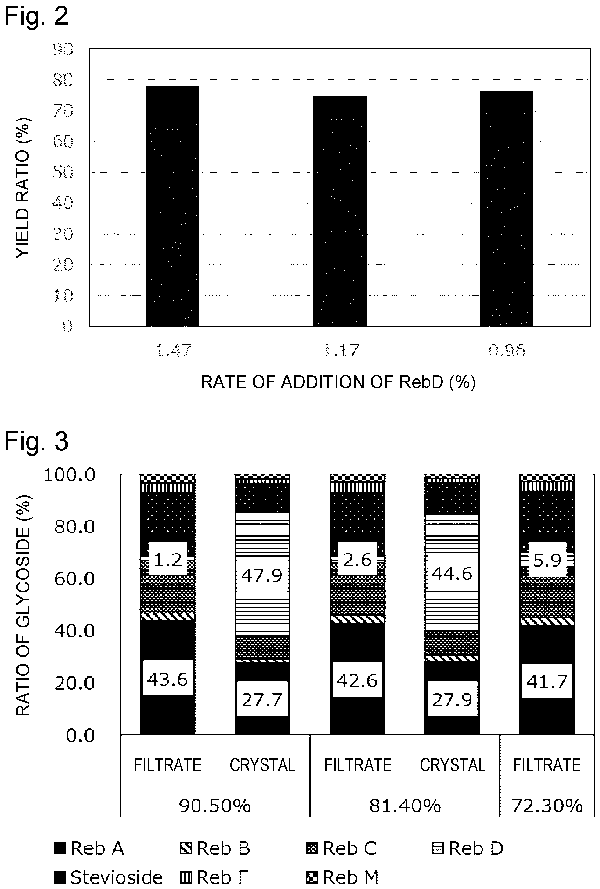

In the formula, C represents the ratio of the amount (excluding the amount of the seed crystal) of RebD added to the solvent (ppm), and C* represents the saturated solubility (ppm). The unit of the amount of the solvent is ml.
Next, a crystal was obtained by performing solid-liquid separation. The filter for use in the solid-liquid separation was 0.45-um membrane filter manufactured by Advantec Co., Ltd.
Thereafter, a RebD crystal was washed with 99% by mass of ethanol. The crystal after washing was dried at 50°C.
The results are illustrated in Figure 2. It was found that a recovery ratio (amount of RebD recovered, relative to the amount of RebD used (including the seed crystal)) of about 75% was exhibited even at any ratio.

<Experimental Example 3>

In the present experiment, the effect of the concentration of ethanol for use as a solvent on the compositional ratio and the yield of TSG (total steviol glycoside) of the RebD crystallized product was studied.

A roughly purified product having the following compositional ratio (unit: % by mass) was prepared. The purity of RebD per TSG in the roughly purified product was 1.8% by mass. The TSG content in the roughly purified product was 59.7% by mass.

[Table 2]

| RebA | RebB | RebC | RebD | Stevioside | REbF | RebM | Others |
|------|------|------|------|-----------|------|------|--------|
| 22. 2 | 1.9 | 7.2 | 1. 1 | 25.2 | 1. 5 | 0. 7 | 40. 3 |

The following amount of the roughly purified product was dissolved in the following concentration and amount of an ethanol solvent (78°C).

Next, cooling was made under the following temperature profile and stirring conditions, to deposit a RebD crystal. A separable round-bottomed flask (manufactured by Tokyo Rikakikai Co., Ltd.) was used in the deposition. Unmodified alcohol (99.9% by mass grade) was used as ethanol, and water was appropriately added thereto to adjust the ethanol concentration. Herein, the ratio of RebD to the solvent was 0.25% by mass, the ratio of TSG to the solvent was 13.8% by mass, the degree $\sigma A$ of supersaturation of RebA (10°C) was 5.10 (in use of a 90.50% by mass ethanol solvent), the degree $\sigma D$ of supersaturation of RebD (10°C) was 24.37 (in use of a 90.50% by mass ethanol solvent), and the $\sigma D/\sigma A$ was 4.78 (in use of a 90.50% by mass ethanol solvent).

The calculation formula of the degree of supersaturation is as follows. The initial concentration (the ratio of the amount of a substance added to the solvent) does not include the amount of the seed crystal.

$$\sigma = \text{(Initial concentration - Saturated solubility at}$$

$$10°C) / \text{(Saturated solubility at } 10°C)$$

[Table 3]

| -Primary crystallization parameters | |
|---|---|
| Sample | Roughly purified product 250g |
| Solvent | 95.0% by mass, 90.5% by mass, 81.40% by mass, 72.30% by mass, EtoH 1, 100ml |
| Liquid temperature (Start) | 78°C |
| Liquid temperature (End) | 10°C |
| Time | 1211 |
| Stirring speed | 600rpm |
| Temperature profile | Controlled |
| Seed crystal | - |

Next, a RebD crystal was obtained by performing solid-liquid separation. The filter for use in the solid-liquid separation was 0.45-um membrane filter manufactured by Advantec Co., Ltd.

Thereafter, a RebD crystal was washed with 99% by mass of ethanol. The RebD crystal after washing was dried at 50°C, and thus a RebD crystallized product was obtained.

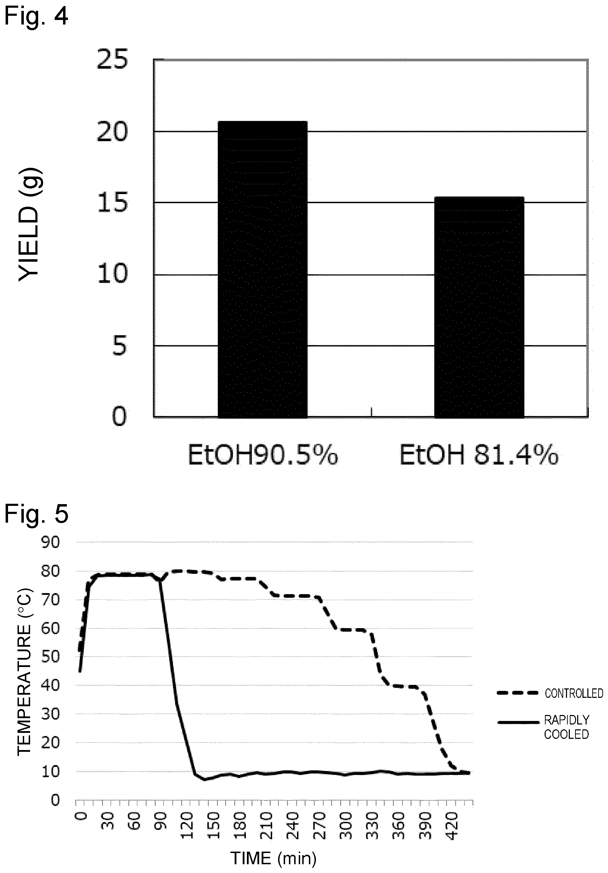

The ratio of glycoside between the RebD crystallized product obtained and the filtrate was analyzed with LCMS (LCMS 8050 manufactured by Shimadzu Corporation). The results are illustrated in Figure 3 and Figure 4.

In the case of use of ethanol having a concentration of 95% by mass under conditions of the present experiment, no solid-liquid separation could be made due to an increase in viscosity of a slurry. On the other hand, in the case of ethanol having a concentration of about 70% by mass, no crystal was precipitated.

While Figure 4 indicated that crystal precipitation tended to be poorly made in the case of a low ethanol concentration, Figure 3 indicated that the purity of RebD was not much affected by the ethanol concentration. It was found that an optimal ethanol solvent concentration was present at a ratio of TSG to the solvent, adopted in the present experiment (13.8%).

<Experimental Example 4>

The cooling rate has a large effect on appearance of a crystal nuclear and crystal growth. For example, in the case of a high cooling rate, consumption of supersaturation is incompletely made and nucleus generation is often predominant, during crystal growth from the initial state of a few nuclei. On the other hand, in the case of a low rate of temperature drop, crystal growth is predominant. However, the crystallizing time tends to be longer in this case, while depends on the consumption rate of supersaturation.

In the present experiment, the effect of the cooling rate on the compositional ratio and yield of TSG was studied.

Specifically, a RebD crystallized product was obtained by performing primary crystallization in the same manner as in Experimental Example 3 except that conditions in the following Table were adopted instead of conditions in Table 3.

[Table 4]

| Primary crystallization parameters | |
| --- | --- |
| Sample | Roughly purified product 250 g |
| Solvent | 90 % by mass EtOH 1,100 ml |
| Liquid temperature (Start) | 80°C |
| Liquid temperature (End) | 10°C |
| Time | 6H |
| Stirring speed | 600rpm |
| Temperature profile | Rapidly cooled, Controlled (See Figure 5) |
| Seed crystal | - |

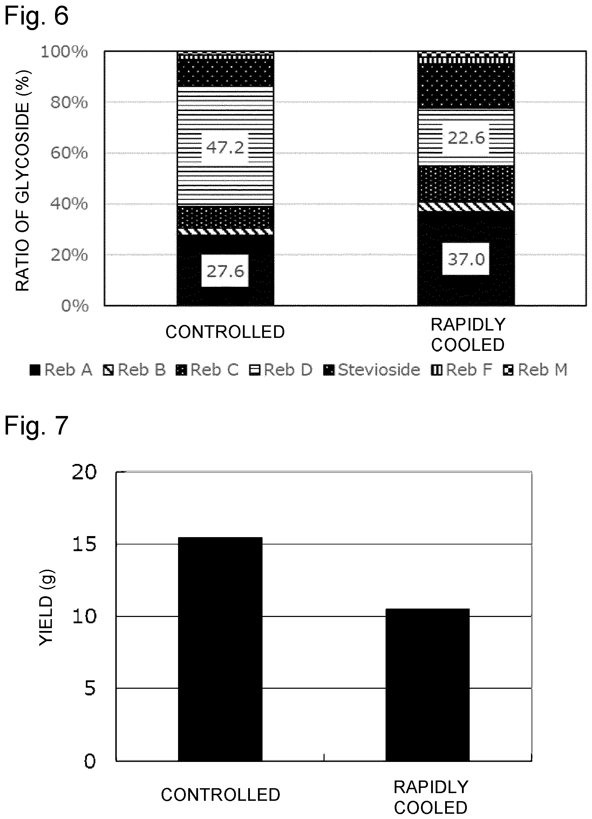

The results are illustrated in Figure 6 and Figure 7. As clear from Figure 6 and Figure 7, a precipitated product lower in purity of RebD relative to TSG was obtained at a higher rate of temperature drop. The yield was also decreased. A precipitated product having viscous properties was obtained at a higher rate of temperature drop. On the other hand, no viscous properties were confirmed at a lower rate thereof.

<Experimental Example 5>

The crystallizing time has a large effect on crystal growth and also has an effect on the time cycle of the entire purification step. The crystallizing time consequently also has an effect on cost and product characteristics (yield, purity, and the like). Thus, the effect of the crystallizing time on the compositional ratio and yield of TSG were studied in the present experiment.

Specifically, a RebD crystallized product was obtained by performing primary crystallization in the same manner as in Experimental Example 3 except that conditions in the following Table were adopted instead of conditions in Table 3.

[Table 5]

| Primary crystallization parameters | |
| --- | --- |
| Sample | Roughly purified product 250 g |
| Solvent | 90 % by mass EtOH 1,100 ml |
| Liquid temperature (Start) | 80°C |
| Liquid temperature (End) | 10°C |
| Time | 6H, 12H |
| Stirring speed | 600rpm |
| Temperature profile | Control 1 ed (See Figure 8) |
| Seed crystal | - |

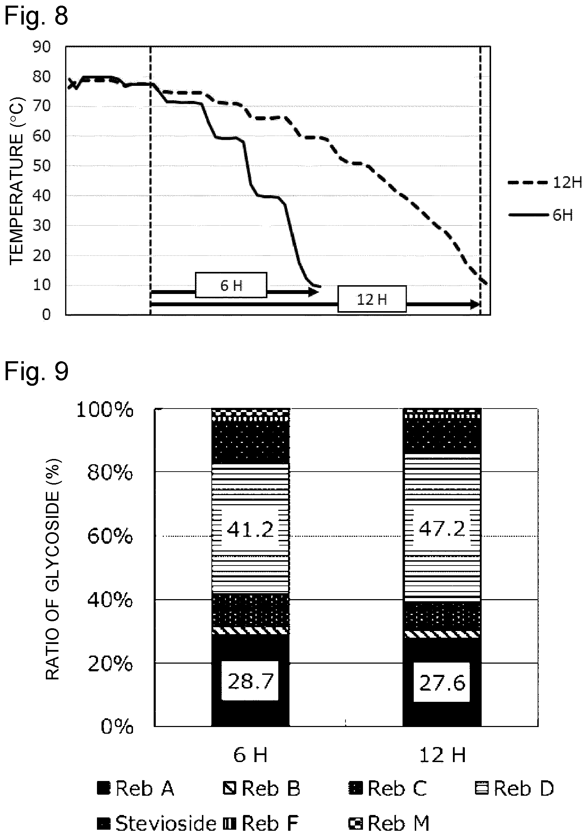

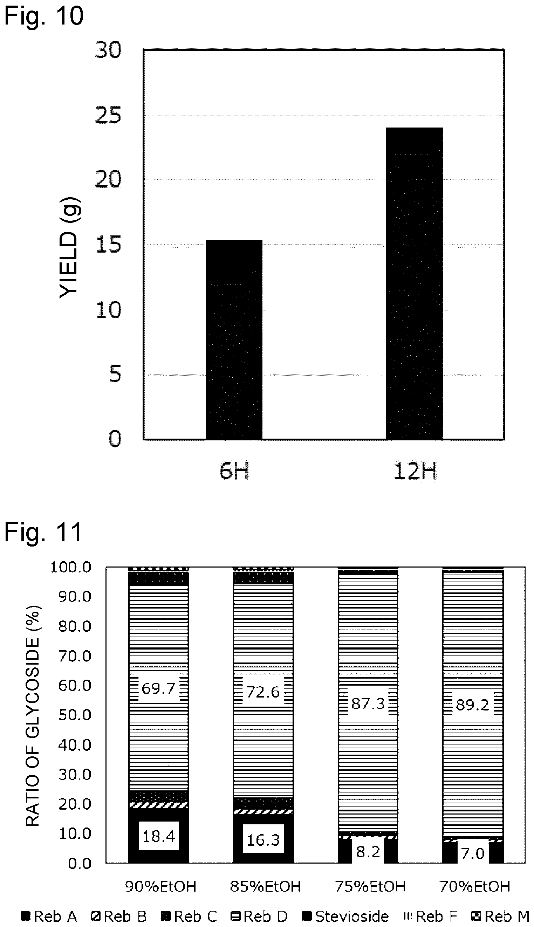

The results are illustrated in Figure 9 and Figure 10. It was found that the crystallizing time, while had no effect on the compositional ratio of glycoside, had an effect on the yield. A crystal was further precipitated from the filtrate after crystallization in the case of 6 H crystallization. It was considered from this that a crystallizing time of at least 12 H or more was needed. It was here considered that further time shortening was possible depending on whether or not the seed crystal was used.

<Experimental Example 6>

In the present experiment, whether or not secondary crystallization was performed with the primary crystallized product was studied for the purpose of a further increase in purity of RebD. Specifically, primary crystallization conditions were determined as shown in the following Table, with reference to the results of Experimental Examples 2 to 5. A primary crystallized product of RebD was then obtained by performing primary crystallization in the same manner as in Experimental Example 3 except that conditions in the following Table were adopted instead of conditions in Table 3.

[Table 6]

| Primary crystallization parameters | |
|---|---|
| Sample | Roughly purified product 250 g |
| Solvent | 90 % by mass EtOH 1,100 ml |
| Liquid temperature (Start) | 80°C |
| Liquid temperature (End) | 10°C |
| Time | 6 H or more |
| Stirring speed | 600rpm |
| Temperature profile (rate of drop) | Controlled |
| Seed crystal | - |

Thereafter, a secondary crystallized product of RebD was obtained by performing secondary crystallization in the same manner as in Experimental Example 3 except that conditions in the following Table were adopted instead of conditions in Table 3. Herein, the ratio of RebD to the solvent was 0.56% by mass. The degree of supersaturation of RebA (10°C) was -0.66 and the degree of supersaturation of RebD (10°C) was 53.6.

[Table 7]

| Secondary crystallization parameters | |
|---|---|
| Sample | Primary crystallized product 15.4 g |
| Solvent | 90.0% by mass, 85% by mass, 75% by mass, 70% by mass EtoH, 1100ml |
| Liquid temperature (Start) | 70°C |
| Liquid temperature (End) | 10°C |
| Time | 12II |
| Stirring speed | 350rpm |
| Temperature profile (rate of drop) | Controlled |
| Seed crystal | - |

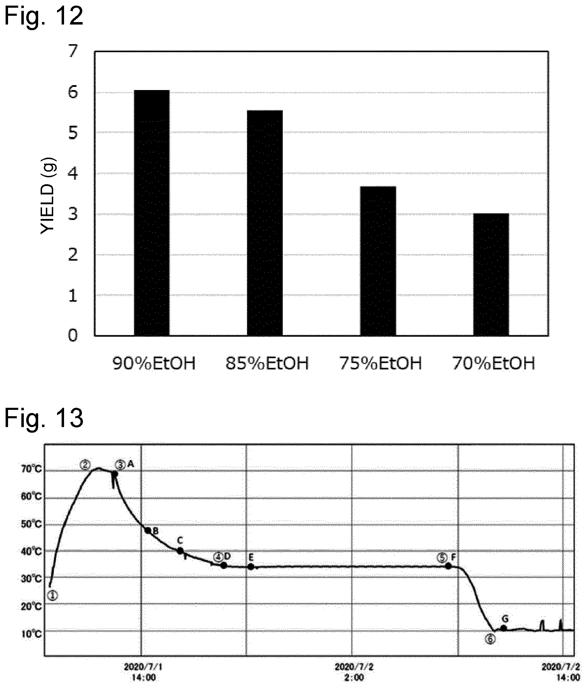

The results were illustrated in Figure 11 and Figure 12. It was found that the yield was decreased by a reduction in ethanol concentration also in secondary crystallization as in the case of studying of the solvent in primary crystallization in Experimental Example 3.

With respect to information on the final compositional ratio and yield ratio of a crystal in the present Experimental Example, the yield ratio was about 43% by mass in the case of operations until secondary crystallization performed under a condition where a purity of about 90% was achieved (namely, the ethanol concentration in secondary crystallization was 70% by mass).

<Experimental Example 7>

In the present Experimental Example, a case was studied where not RebD, but RebA was crystallized in primary crystallization.

An extraction operation was performed with a dry leaf of a stevia plant, and the resulting extract was subjected to a solid-liquid separation operation. The content ratio of each steviol glycoside in the dry leaf (unit: % by mass) was as follows. The content of TSG in the dry leaf used in the present Experimental Example was 16.6 g per 100 g.

[Table 8]

| Reb A | Reb B | Reb C | Reb D | Stevioside | Reb F | Reb M | Others |
|-------|-------|-------|-------|-----------|-------|-------|--------|
| 11. 7 | 0.1 | 1.0 | 0.6 | 2.6 | 0.3 | 0.3 | 83.4 |

The clarified liquid after solid-liquid separation was subjected to steps of flocculation, purification with the resin, and evaporation concentration. As a result, a roughly purified product having a compositional ratio (unit: % by mass) shown in the following Table was obtained. The proportion of RebD per TSG in the roughly purified product was 3.8% by mass.

[Table 9]

| RebA | RebB | RebC | RebD | Stevioside | RebF | RebM |
|------|------|------|------|-----------|------|------|
| 69. 5 | 0.4 | 5. 3 | 3. 8 | 18.3 | 1. 8 | 1. 5 |

The following amount of the roughly purified product was dissolved in the following concentration and amount of an ethanol solvent (40°C). The rate of addition of RebD to the solvent was 0.22% by mass.

Next, a RebA crystal was deposited by cooling under the following temperature profile and stirring conditions. A separable round-bottomed flask (manufactured by Tokyo Rikakikai Co., Ltd.) was used in the deposition. Unmodified alcohol (99.9% by mass grade) was used as ethanol.

Next, a RebA crystal was obtained by performing solid-liquid separation. The filter for use in the solid-liquid separation was 0.45-um membrane filter manufactured by Advantec Co., Ltd.

Thereafter, a RebA crystal was washed with 99% by mass of ethanol. The RebA crystal after washing was dried at 50°C.

[Table 10]

| Primary crystallization parameters | |
|---|---|
| Sample | Roughly purified product 25.8 g |
| Solvent | 99% by mass EtOH 70 ml |
| Liquid temperature (Start) | 40°C |
| Liquid temperature (End) | 4°C |
| Time | 18H |
| Stirring speed | 350rpm |
| Temperature profile | Controlled |
| Seed crystal | - |

The primary crystallized product was analyzed with LCMS 8050 manufactured by Shimadzu Corporation. Table 11 below shows the ratio of each steviol glycoside to TSG in the primary crystallized product (unit: % by mass). Table 12 describes information on the yield ratio (unit: % by mass). The purity of RebA relative to TSG was 98.6% and the yield ratio of RebA was 41.8%. As a result, about 11.5% by mass of the yield of RebD was incorporated into a RebA crystal.

[Table 11]

| RebA | RebB | RebC | RebD | Stevioside | RebF | RebM |
|------|------|------|------|------------|------|------|
| 98. 6 | 0 | 0 | 1. 4 | 0 | 0 | 0 |

[Table 12]

| | RebA | RebD |
|---|------|------|
| Primary crystallized product (RebA crystallization) | 41. 80 | 11.51 |

Next, RebD crystallization from the mother liquid of primary crystallization was tried with a RebD seed crystal. The purity of RebD relative to TSG was 94% by mass or more, as shown in the following Table.

[Table 13]

| RebA | RebB | RebC | RebD | Stevioside | RebF | RebM |
|------|------|------|------|------------|------|------|
| 4. 1 | 0.2 | 0.3 | 94. 1 | 1.4 | 0.0 | 0.0 |

<Experimental Example 8>

In the present experiment, optimized conditions obtained as the results of the small-scale experiment performed in a laboratory were adopted to perform RebD crystallization.

An extraction operation was performed with a dry leaf of a stevia plant, and the resulting extract was subjected to a solid-liquid separation operation. The content ratio of each steviol glycoside in the dry leaf was as follows. The content of TSG in the dry leaf used in the present Experimental Example was 8.7 g per 100 g.

[Table 14]

| Reb A | Reb B | Reb C | Reb D | Stevioside | Reb F | Reb M | Others |
|-------|-------|-------|-------|------------|-------|-------|--------|
| 6. 2 | 0. 1 | 0.4 | 0. 7 | 0.6 | 0.2 | 0.5 | 91. 3 |

The clarified liquid after solid-liquid separation was subjected to steps of flocculation, purification with the resin, and evaporation concentration. As a result, a roughly purified product having a compositional ratio (unit: % by mass) shown in the following Table was obtained. The proportion of RebD per TSG in the roughly purified product was 4.6% by mass. The proportion of TSG in the roughly purified product was 54.0% by mass.

[Table 15]

| RebA | RebB | RebC | RebD | Stevioside | RebF | RebM | Others |
|------|------|------|------|------------|------|------|--------|
| 39. 0 | 0 | 2.3 | 2. 5 | 8. 2 | 1. 2 | 0. 78 | 46. 0 |

The following amount of the roughly purified product (represented as a sample in the Table) was dissolved in the following concentration and amount of an ethanol solvent (40°C). Herein, the ratio of TSG to the solvent was 12.5% by mass, the ratio of RebD to the solvent was 0.58% by mass, the degree of supersaturation of RebA (10°C) was 9.98, and the degree of supersaturation of RebD (10°C) was 57.64.

Next, crystallization was made by cooling under the following temperature profile and stirring conditions. A separable round-bottomed flask (manufactured by Tokyo Rikakikai Co., Ltd.) was used in the crystallization. Unmodified alcohol (99.9% by mass grade) was used as ethanol, and water was appropriately added thereto to adjust the ethanol concentration.

Next, a crystal was obtained by performing solid-liquid separation. The filter for use in the solid-liquid separation was 0.45-um membrane filter manufactured by Advantec Co., Ltd.

Thereafter, a crystal was washed with 99% by mass of ethanol. The crystal after washing was dried at 50°C.

[Table 16]

| Primary crystallization parameters | |
| --- | --- |
| Sample | 18. 6g |
| Solvent | 90% by mass EtOH 80 ml |
| Liquid temperature (Start) | 40°C |
| Liquid temperature (End) | 17°C |
| Time | 18H |
| Stirring speed | 350rpm |
| Temperature profile | Controlled |
| Seed crystal | - |

As a result of primary crystallization, 343 mg of a crystal could be obtained which had a purity of TSG (the proportion of TSG per crystallized product) of 76.2% and a purity of RebD (the proportion of RebD per TSG) of 70.5%. The yield ratio (yield ratio per unit step) was 39.5%.

<Experimental Example 9>

An extraction operation was performed with a dry leaf of a stevia plant, and the resulting extract was subjected to a solid-liquid separation operation. The proportion of each steviol glycoside per TSG in the dry leaf (% by mass) was as described below. The content of TSG in the dry leaf used in the present Experimental Example was 11.8 g per 100 g.

[Table 17]

| Reb A | Reb B | Reb C | Reb D | Stevioside | Reb F | Reb M |
| --- | --- | --- | --- | --- | --- | --- |
| 76. 1 | 0.9 | 4.4 | 6.9 | 3.6 | 2.4 | 5.8 |

The clarified liquid after solid-liquid separation was subjected to steps of flocculation, purification with the resin, and evaporation concentration. The resulting steviol glycoside composition was spray dried, to obtain a roughly purified product. The compositional ratio of the roughly purified product after the spray drying is as follows.

[Table 18]

| RebA | RebB | RebC | RebD | Stevioside | RebF | RebM | Others |
| --- | --- | --- | --- | --- | --- | --- | --- |
| 41.0 | 0.3 | 2.9 | 3. 7 | 3. 7 | 1.4 | 2.9 | 44.0 |

Unmodified alcohol (99.9% by mass grade) was used, and water was appropriately added thereto to adjust ethanol having the following concentration and amount.

A crystallization container (having a volume of 1 L) equipped with a stirrer was loaded with an ethanol solvent and heated.

When the temperature of the ethanol solvent reached about 55°C, the container was loaded with the roughly purified product.

The temperature was dropped with stirring at about 350 rpm. Cooling was here evenly made for the cooling time (namely, 11 hours) - 1 hour (namely, 10 hours).

After completion of crystallization, solid-liquid separation was performed. The filter for use in the solid-liquid separation was 0.45-um membrane filter manufactured by Advantec Co., Ltd.

The resulting crystal was dried at 60 to 70°C.

[Table 19]

| Primary crystallization parameters | |
| --- | --- |
| Ratio of TSG to solvent | 12.8% by mass |
| Solvent | 90% by mass EtOH 482 ml |

(continued)

| Primary crystallization parameters | |
|---|---|
| Liquid temperature (Start) | 55°C |
| Liquid temperature (End) | 17°C |
| Time | 11H |
| Stirring speed | 350rpm |
| Rate of temperature drop | Controlled |
| Seed crystal | - |

The yield ratio per unit step (the ratio of RebD crystallized to RebD included in the roughly purified product before crystallization) was 80.0% by mass. The purity of TSG (the ratio of TSG to the crystallized product) was 63.2% by mass and the purity of RebD (the ratio of RebD to TSG) was 35.8% by mass.

<Experimental Example 10>

The present experiment studied about RebD crystallization in the case of a large amount of RebA present in the ethanol solvent, namely, in the case of a high degree of supersaturation of RebA set.

1. Extraction·solid-liquid separation

Ion-exchange water in an amount of 15 times (mass) relative to a stevia dry leaf (water content ratio: 3 to 4% by weight) was heated to 60°C $\pm$ 5°C, and the stevia dry leaf was immersed in the water. Thereafter, extraction was performed with a kneader extractor (SKN-R100, manufactured by Sanyukiki Co., Ltd.) for 60 minutes, with stirring by a stirring bar at 8 rpm. Next, filtration was made by passing through 18-mech and 140-mech, cooling was made with cold water in a heat exchanger, and the filtrate was subjected to solid-liquid separation in a disk-type centrifuge (9150 rpm (11601 G), 24 L/min), to obtain a primary extraction liquid. In the meantime, the leaf after the filtration was extracted again in the same conditions and subjected to solid-liquid separation to obtain a transparent secondary extraction liquid, and the extraction liquid was added to the primary extraction liquid to obtain a clarified liquid.

The proportion of each steviol glycoside per TSG in the dry leaf was measured according to a liquid chromatography mass spectrometry method (LC/MS/MS) (LCMS 8050 manufactured by Shimadzu Corporation). The results are shown in the following Table (unit: % by mass).

[Table 20]

| Reb A | Reb B | Reb C | Reb D | Stevioside | Reb F | Reb M |
|---|---|---|---|---|---|---|
| 68. 3 | 0. 6 | 4. 2 | 10. 2 | 10. 2 | 1.8 | 4.8 |

2. Flocculation

Ca(OH)$_2$ (calculated from Brix (soluble solid content concentration)) was added to the clarified liquid in an amount corresponding to 16.16% by mass of the soluble solid content in the clarified liquid, and the resulting mixed liquid was stirred for 15 minutes. Thereafter, FeCl$_3$·6H$_2$O was added in an amount corresponding to 28.28% by mass of the soluble solid content in the clarified liquid, the mixed liquid was stirred for 30 minutes and the pH was adjusted to 7 by citric acid, and thereafter a 0.5% (w/v) chitosan solution was added in a volume (mL) corresponding to 5.63 times the soluble solid content (g) in the clarified liquid. The mixed liquid was strongly stirred for 3 minutes and weakly stirred for 2 minutes, and then left to still stand for 10 minutes. Thereafter, a solidified precipitated product electrically neutral was removed by centrifugation. As a result, a clear treatment liquid was obtained.

3. Purification with resin

In the purification with the resin, performed were (i) purification with an anion exchange resin and (ii) purification with a hydrophobic porous resin (with no ion exchange group introduced).

(i) Purification with anion exchange resin

A column was packed with a highly porous basic anion exchange resin (manufactured by Mitsubishi Chemical Corporation), and the column was loaded with the treatment liquid after flocculation and separation, to perform purification. The column was loaded with the treatment liquid after flocculation and separation, and then subjected to pushing out with ion-exchange water in a volume of twice the volume of the column, and a solution including a steviol glycoside composition purified was recovered. Black impurities and a colored component in the treatment liquid were removed by this purification.

(ii) Purification with hydrophobic porous resin

A column was packed with a hydrophobic porous resin (manufactured by Mitsubishi Chemical Corporation), and the column was loaded with a specimen after (i) purification with an anion exchange resin, to perform purification. The hydrophobic porous resin here used was one which was a copolymer of styrene and divinylbenzene, which had no ion exchange group, and which had a most frequent pore radius of 45 Å. The column was loaded with the solution after (i) purification described above, and thereafter the column was washed with an aqueous 0.01 M citric acid solution in a volume of 3 times the volume of the column and an aqueous 0.01 M sodium hydroxide solution in a volume of 3 times the volume of the column. Thereafter, a steviol glycoside composition was eluted and recovered with an aqueous 60% ethanol solution in a volume of 4 times the volume of the column.

4. Evaporation concentration

A centrifugal thin film vacuum evaporator Evapol (manufactured by Okawara Mfg. Co., Ltd.) was used to remove ethanol with evaporation concentration of a solution. Water remained even after the evaporation concentration treatment, and the composition was in the form of a liquid.
The resulting steviol glycoside composition was spray dried, to obtain a roughly purified product. The compositional ratio of the roughly purified product after the spray drying (unit: % by mass) is as follows.

[Table 21]

| Reb A | Reb B | Reb C | Reb D | Stevioside | Reb F | Reb M | Others |
|-------|-------|-------|-------|------------|-------|-------|--------|
| 67. 0 | 0.0 | 4. 1 | 7.5 | 11.5 | 1.6 | 3. 3 | 5. 1 |

Crystallization was performed in the same manner as in Experimental Example 9 except that conditions in the following Table were adopted. The results were shown in Table 31 below.

[Table 22]

| Primary crystallization parameters | |
|---|---|
| Solvent | 90% by mass EtOH 734.8 ml |
| Ratio of RebD to solvent | 1.6% by mass |
| Liquid temperature (Start) | 55°C |
| Liquid temperature (End) | 10°C |
| Time | 11H |
| Stirring speed | 350rpm |
| Temperature profile | Controlled |
| Seed crystal (RebD) | Cs=0.08 |

<Experimental Example 11>

Extraction and solid-liquid separation were performed in the same manner as in Experimental Example 10. The proportion of each steviol glycoside per TSG in the dry leaf (% by mass) was measured in the same manner as in Experimental Example 10, and was as shown in the following Table.

[Table 23]

| Reb A | Reb B | Reb C | Reb D | Stevioside | Reb F | Reb M |
|---|---|---|---|---|---|---|
| 68.4 | 1.0 | 5.4 | 9.2 | 7.8 | 1.5 | 6. 6 |

Steps of flocculation, purification with the resin, and evaporation concentration were performed in the same manner as in Experimental Example 10. The resulting steviol glycoside composition was spray dried, to obtain a roughly purified product. The compositional ratio of the roughly purified product after the spray drying (% by mass) is as follows.

[Table 24]

| Reb A | Reb B | Reb C | Reb D | Stevioside | Reb F | Reb M | Others |
|---|---|---|---|---|---|---|---|
| 64.8 | 0.6 | 5. 5 | 8.1 | 9.1 | 1.3 | 4. 7 | 6.0 |

Primary crystallization was performed in the same manner as in Experimental Example 9 except that conditions in the following Table were adopted.

[Table 25]

| Primary crystallization parameters | |
|---|---|
| Solvent | 90% by mass EtOH 1,117 ml |
| Ratio of RebD to solvent | 1.6% by mass |
| Liquid temperature (Start) | 55°C |
| Liquid temperature (End) | 10°C |
| Time | 7.5ll |
| Stirring speed | 350rpm |
| Temperature profile | Controlled |
| Seed crystal(RebD) | Cs=0. 08 |

Secondary crystallization was performed in the same manner as in the primary crystallization except that conditions in the following Table were adopted. The results were shown in Tables 31 to 33 below.

[Table 26]

| Secondary crystallization parameters | |
|---|---|
| Solvent | 90% by mass EtOH 715 ml |
| Ratio of RebD to solvent | 2.2% by mass |
| Liquid temperature (Start) | 55°C |
| Liquid temperature (End) | 10°C |
| Time | 24H |
| Stirring speed | 350rpm |
| Temperature profile | Controlled |
| Seed crystal (RebD) | Cs=0. 08 |

<Experimental Example 12>

Extraction and solid-liquid separation were performed in the same manner as in Experimental Example 10. The proportion of each steviol glycoside per TSG in the dry leaf (% by mass) was measured in the same manner as in Experimental Example 10, and was as shown in the following Table.

[Table 27]

| Reb A | Reb B | Reb C | Reb D | Stevioside | Reb F | Reb M |
|---|---|---|---|---|---|---|
| 53. 6 | 1.2 | 4.2 | 20.5 | 11.2 | 1. 1 | 8.2 |

Steps of flocculation, purification with the resin, and evaporation concentration were performed in the same manner as in Experimental Example 10. The resulting steviol glycoside composition was spray dried, to obtain a roughly purified product. The compositional ratio of the roughly purified product after the spray drying (% by mass) is as follows

[Table 28]

| Reb A | Reb B | Reb C | Reb D | Stevioside | Reb F | Reb M | Others |
|---|---|---|---|---|---|---|---|
| 50. 8 | 0.4 | 4.7 | 15. 3 | 13. 8 | 1. 1 | 5. 9 | 8. 1 |

Crystallization was performed in the same manner as in Experimental Example 9 except that conditions in the following Table were adopted. The degree of supersaturation of RebA (10°C) was 8.2 and the degree of supersaturation of RebD (10°C) was 160.1.

[Table 29]

| Primary crystallization parameters | |
|---|---|
| Solvent | 90% by mass EtOH 1,538 ml |
| Ratio of RebD to solvent | 1.6% by mass |
| Liquid temperature (Start) | 55°C |
| Liquid temperature (End) | 10°C |
| Time | 7. 5H |
| Stirring speed | 200rpm |
| Temperature profile | Controlled |
| Seed crystal (RebD) | Cs=0. 08 |

Secondary crystallization was performed in the same manner as in the primary crystallization except that conditions in the following Table were adopted. The results are shown in Table 32 and Table 33.

[Table 30]

| Secondary crystallization parameters | |
|---|---|
| Amount of roughly purified product added | About 42g |
| Solvent | 90% by mass EtOH 715 ml |
| Ratio of RebD to solvent | 3.4% by mass |
| Liquid temperature (Start) | 55°C |
| Liquid temperature (End) | 10°C |
| Time | 24H |
| Stirring speed | 200rpm |
| Temperature profile | Controlled |
| Seed crystal | - |

The following Tables show the purity and the degree of supersaturation of each component, and the yield ratio of RebA per unit step-yield ratio of RebD per unit step, in the roughly purified products used in Experimental Examples 10 to 12.

The purity of RebA and the purity of RebD were each measured with LCMS 8050 manufactured by Shimadzu Corporation, and represented as the ratio (w/w) to TSG.

The calculation formula of the degree of supersaturation is as described above. In calculation of the degree of supersaturation, the respective saturated solubilities (90% by mass of EtOH, 10°C) of RebA and RebD were as follows.

RebA = 8270.5 ppm

RebD = 99.33 ppm

The yield ratio of RebA per unit step represents the ratio of the amount of RebA included in the crystallized product to the amount of RebA in the raw material used in crystallization. The same applies to the yield ratio of RebD per unit step.

[Table 31]

| Experimental Example | Purity of RebA before primary crystallization (% by mass) | Purity of RebD before primary crystallization (% by mass) | Purity of TSG before primary crystallization (% by mass) | Degree of super-saturation σ(Re-bA) before pri-mary crystallization | Degree of super-saturation σ (RebD) before primary crystallization | σ(D)/σ(A) | Purity of RebA after primary crystallization (% by mass) | Purity of RebD after primary crystallization (% by mass) | Yield ratio of RebA per unit in primary crystallization (%) | Yield ratio of RebD per unit in primary crystallization (%) |
|---|---|---|---|---|---|---|---|---|---|---|
| 10 | 67.1 | 7.5 | 94.89 | 18.54 | 179.9 | 9.70 | 78.3 | 13.5 | 52.6 | 84.2 |
| 11 | 64.8 | 8.1 | 94 | 14.7 | 161.2 | 11.0 | 26.0 | 63.4 | 4.1 | 82.5 |

[Table 32]

| Experimental Example | Purity of before crystallization (%) RebA | Purity of secondary before crystallization (%) | RebD Purity secondary before (% by | of TSG Degree of secondary supersaturation crystallization σ (RebA) at mass) 10°C | Degree of supersaturation σ(RebD) at 10°C | σ(D)/σ(A) | Purity of RebA after secondary crystallization (%) | Purity of RebD after secondary crystallization (%) | Yield ratio of RebD per unit step (%) |
|---|---|---|---|---|---|---|---|---|---|
| 11 | 26.0 | 63.4 | 109.5 | 0.00027 | 202.348 | 749,437.0 | 4.0 | 92.2 | 97.7 |
| 12 | 49.4 | 14.8 | 112.8 | 6.2 | 179.6 | 29.0 | 11.5 | 84.9 | 85.7 |

[Table 33]

| | Experimental Example | Solvent | Amount of solvent (ml) | Seed crystal (mg) | Temperature (initial) | Temperature (end) | Time (H) | Purity of RebD (%) |
|---|---|---|---|---|---|---|---|---|
| First crystallization | 11 | 90% EtOH | 1117.1 | 1240 | 55 | 10 | 7.5 | 63.4 |
| | 12 | 90% EtOH | 1538.6 | 1957 | 55 | 10 | 7.5 | 55.1 |
| Second crystallization | 11 | 90% EtOH | 715 | 1149 | 55 | 10 | 24 | 92.2 |
| | 12 | 90% EtOH | 715 | - | 55 | 10 | 24 | 85.0 |

It was presumed that precipitation of RebA, while depended on the degree of supersaturation of RebD, was increased with a degree of supersaturation of RebA of 15 to 18 as the boundary. While some purities were more than 100% in the above Tables, the reason for this was considered because the weight was increased due to the remaining solvent or there was any analysis error.

<Experimental Example 13>

The present experiment studied about whether or not the compositional ratio of a RebD crystallized product was changed in use of an ethanol solvent as the solvent for crystallization, as compared with that in use of a methanol solvent.

Two kinds of RebD crystallized products were obtained. Both the products were considered to be crystallized with a methanol solvent. Sample No. 1 was a RebD crystallized product manufactured by GLG, and sample No. 2 was a RebD crystallized product manufactured by JNRJ.

The secondary crystallized product of RebD obtained in Experimental Example 11 was adopted as sample No. 3.

Extraction and solid-liquid separation were performed in the same manner as in Experimental Example 10. The proportion of each steviol glycoside per TSG in the dry leaf (% by mass) was measured in the same manner as in Experimental Example 10, and was as shown in the following Table.

[Table 34]

| Reb A | Reb B | Reb C | Reb D | Stevioside | Reb F | Reb M |
|---|---|---|---|---|---|---|
| 66. 9 | 1. 1 | 5. 1 | 9.6 | 12. 5 | 1.3 | 3.4 |

Steps of flocculation, purification with the resin, and evaporation concentration were performed in the same manner as in Experimental Example 10. The resulting steviol glycoside composition was spray dried, to obtain a roughly purified product. The compositional ratio of the roughly purified product after the spray drying (% by mass) is as follows.

[Table 35]

| Reb A | Reb B | Reb C | Reb D | Stevioside | Reb F | Reb M | Others |
|---|---|---|---|---|---|---|---|
| 55. 8 | 0. 1 | 0. 0 | 6. 6 | 11. 1 | 1. 1 | 2. 4 | 22. 9 |

Primary crystallization was performed in the same manner as in Experimental Example 9 except that conditions in the following Table were adopted. The degree of supersaturation of RebA (10°C) was 16.1 and the degree of supersaturation of RebD (10°C) was 160.2.

[Table 36]

| Primary crystallization parameters | |
|---|---|
| Solvent | 90% by mass EtOH 819 ml |
| Ratio of RebD to solvent | 1.6% by mass |
| Liquid temperature (Start) | 55°C |
| Liquid temperature (End) | 10°C |
| Time | 6H |
| Stirring speed | 200rpm |
| Temperature profile | Control 1 ed |
| Seed crystal (RebD) | Cs=0. 08 |

Secondary crystallization was performed in the same manner as in the primary crystallization except that conditions in the following Table were adopted.

[Table 37]

| Secondary crystallization parameters | |
|---|---|
| Solvent | 90% by mass EtOH 715 ml |
| Ratio of RebD to solvent | 1.94% by mass |
| Liquid temperature (Start) | 55°C |
| Liquid temperature (End) | 10°C |
| Time | 24ll |
| Stirring speed | 200rpm |
| Temperature profile | Controlled |
| Seed crystal | - |

The secondary crystallized product of RebD obtained was adopted as sample No. 4.

[Table 38]

| Purity of RebA before secondary crystallization (%) | Purity of RebD before secondary crystallization (%) | Purity of TSG before secondary crystallization (%) | Degree of supersaturation σ (RebA)tion at 10°C | Degree of supersaturaσ (RebD) at 10°C | σ(D)/σ (A) | Purity of RebA after secondary crystallization (%) | Purity of RebD after secondary crystallization (%) | Yield ratio of RebD per unit step (%) |
|---|---|---|---|---|---|---|---|---|
| 25.9 | 57.8 | 115 | 0.04943 | 193.81 | 3,920.8 | 8.3 | 90.6 | 86.7 |

The secondary crystallized product of RebD obtained in Experimental Example 12 was adopted as sample No. 5.

One obtained by further drying sample No. 4 at 60 to 70°C for 3 days was adopted as sample No. 6.

One obtained by further drying sample No. 5 at 60 to 70°C for 3 days was adopted as sample No. 7.

Each of the samples was dissolved in dimethyformamide one by one, and the methanol content and the ethanol content were measured with HS (head space)-GC/MS. The results are shown in the following Table.

[Table 39]

| No. | Additional drying | MeOII (ppm) | EtOII (ppm) | MeOH/ EtOH | Minimum value of MeOH/EtOH expected* |
|---|---|---|---|---|---|
| 1 | | 5 | 4000 | 0.00125 | 0.00100 |
| 2 | | 30 | 1500 | 0. 02000 | 0.00600 |
| 3 | No | 0.9 less than minimum determination limit | 4200 | 0. 00021 | |
| 4 | | 9 | 13000 | 0. 00069 | |
| 5 | | 1.8 less than minimum determination limit | 11000 | 0. 00016 | |
| 6 | Yes | 4 | 9500 | 0. 00042 | |
| 7 | | 3 | 6500 | 0. 00046 | |
| *Calculated by considering that the upper limit of usual EtOH according to quality standard is 5000 ppm | | | | | |

<Experimental Example 14>

1. Extraction·solid-liquid separation

Water in an amount of 15 times (mass) relative to a stevia dry leaf (water content ratio: 9 to 11% by weight) was heated to 60 to 65°C, and the stevia dry leaf was immersed in the water. Thereafter, extraction was performed for 60 minutes, with stirring at 75 rpm by use of a stirring blade (27 cm radius × 2 blades × 2 stages) in a stirring tank (volume: 400 L). Next, a mixture of the stevia dry leaf and the water was filtered by passing through a mesh (100-mesh) made of nylon installed in a Buchner funnel having a diameter of 95 cm, diatomaceous earth (Celite 503) was added, filtration was made by filter pressing, and filtration with a microfiltration membrane (pore size: 10 μm) was made for solid-liquid separation, to obtain a primary extraction liquid. In the meantime, the leaf after the filtration was extracted again in the same conditions and subjected to solid-liquid separation to obtain a transparent secondary extraction liquid, and the extraction liquid was added to the primary extraction liquid to obtain a clarified liquid.

2. Flocculation

$Ca(OH)_2$ (calculated from Brix (soluble solid content concentration)) was added to the clarified liquid in an amount corresponding to 16.16% by mass of the soluble solid content in the clarified liquid, and the resulting mixed liquid was stirred for 15 minutes. Thereafter, $FeCl_3·6H_2O$ was added in an amount corresponding to 28.28% by mass of the soluble solid content in the clarified liquid, the mixed liquid was stirred for 30 minutes and the pH was adjusted to 7 by citric acid, and thereafter a 0.5% (w/v) chitosan solution was added in a volume (mL) corresponding to 5.63 times the soluble solid content (g) in the clarified liquid. The mixed liquid was strongly stirred for 3 minutes and weakly stirred for 2 minutes, and then left to still stand for 10 minutes. Thereafter, a solidified precipitated product electrically neutral was removed by centrifugation. As a result, a clear treatment liquid was obtained.

3. Purification with resin

In the purification with the resin, performed were (i) purification with an anion exchange resin and (ii) purification with a hydrophobic porous resin (with no ion exchange group introduced).

(i) Purification with anion exchange resin

A column was packed with a highly porous basic anion exchange resin (manufactured by Mitsubishi Chemical Corporation), and the column was loaded with the treatment liquid after flocculation and separation, to perform purification. The column was loaded with the treatment liquid after flocculation and separation, and then subjected to pushing out with ion-exchange water in a volume of twice the volume of the column, and a solution including a steviol glycoside composition purified was recovered. Black impurities and a colored component in the treatment liquid were removed by this purification.

(ii) Purification with hydrophobic porous resin

A column was packed with a hydrophobic porous resin (manufactured by Mitsubishi Chemical Corporation), and the column was loaded with a specimen after (i) purification with an anion exchange resin, to perform purification. The hydrophobic porous resin here used was one which was a copolymer of styrene and divinylbenzene, which had no ion exchange group, and which had a most frequent pore radius of 45 Å. The column was loaded with the solution after (i) purification described above, and thereafter the column was washed with an aqueous 0.01 M citric acid solution in a volume of 3 times the volume of the column and an aqueous 0.01 M sodium hydroxide solution in a volume of 3 times the volume of the column. Thereafter, a steviol glycoside composition was eluted and recovered with an aqueous 60% ethanol solution in a volume of 6 times the volume of the column.

4. Evaporation concentration

A centrifugal thin film vacuum evaporator Evapol (manufactured by Okawara Mfg. Co., Ltd.) was used to remove ethanol with evaporation concentration of a solution. Next, an evaporator was used to perform concentration separately at two stages, thereby removing ethanol. Water remained even after the evaporation concentration treatment, and the composition was in the form of a liquid.
The resulting steviol glycoside composition was spray dried, to obtain a roughly purified product. The compositional ratio of the roughly purified product after the spray drying (unit: % by mass) is as follows.

[Table 40]

| Reb A | Reb B | Reb C | Reb D | Stevioside | Reb F | Reb M | Others |
|---|---|---|---|---|---|---|---|
| 23.02% | 0.55% | 2.75% | 25.45% | 15.29% | 0.68% | 13.52% | 18.73% |

Unmodified alcohol (99.9% by mass grade) was used, and water was appropriately added thereto to adjust ethanol having the following concentration and amount.
A separable flask was loaded with an ethanol solvent, and heated.
When the temperature of the ethanol solvent reached about 71°C, the container was loaded with the roughly purified product and a seed crystal. Herein, the degree of supersaturation of RebD (10°C) was 162.854 and the degree of supersaturation of RebA (10°C) was 0.793.
The temperature was decreased with stirring.
After completion of crystallization, solid-liquid separation was performed. The filter for use in the solid-liquid separation was 0.45-um membrane filter manufactured by Advantec Co., Ltd.
The resulting crystal was dried at 60 to 70°C.
Herein, the rate of addition of the seed crystal (Cs) was 0.08.

[Table 41]

| Primary crystallization parameters | |
|---|---|
| Solvent | 90% by mass EtOH 35.2 L |
| Ratio of RebD to solvent | 1.6 w/v% |
| Liquid temperature (Start) | 71°C |
| Liquid temperature (End) | 9°C |
| Time | 7H |
| Temperature profile | Not controlled |
| Seed crystal (RebD) | 45.8g |

The proportion of each steviol glycoside per TSG (here including RebA, RebB, RebC, RebD, stevioside, RebF, RebM, RebN, dulcoside A, RebI, RebG, rubusoside, steviobioside, and RebE) in the primary crystallized product (% by mass) was as shown in the following Table. The yield ratio of RebD per unit step until primary crystallization was 86.39% by mass. The proportion of TSG in the primary crystallized product was 97.4% by mass.

[Table 42]

| Reb A | Reb B | Reb C | Reb D | Stevioside | Reb F | Reb M |
|---|---|---|---|---|---|---|
| 9.69% | 1.14% | 0.54% | 78.08% | 3.12% | 0.14% | 4.30% |

| RebN | DuicosideA | Reb I | Reb G | Rubsoside | steviobioside | Reb E |
|---|---|---|---|---|---|---|
| 1.60% | 0.00% | 0.05% | 0.01% | 0.01% | 0.08% | 1.22% |

Secondary crystallization was performed in the same manner as in the primary crystallization except that conditions in the following Table were adopted. The detail of the temperature profile is illustrated in Figure 13. Herein, the degree of supersaturation of RebD (10°C) was 342.254 and the degree of supersaturation of RebA (10°C) was -0.47.

[Table 43]

| Secondary crystallization parameters | |
|---|---|
| Solvent | 90% by mass EtOH 19.1 L |
| Amount of primary crystallized product added | 860g |
| Ratio of RebD to solvent | 3. 41 w/v% |
| Liquid temperature (Start) | 70°C |
| Liquid temperature (End) | 10°C |
| Time | 24H |
| Temperature profile | Not controlled |
| Seed crystal (RebD) | 52. 3g |

The proportion of each steviol glycoside per TSG in the secondary crystallized product was as shown in the following Table. The yield ratio of RebD per unit step until secondary crystallization was 98.9% by mass. The proportion of TSG in the secondary crystallized product was 95.9% by mass.

[Table 44]

| RebA | Reb B | RebC | RebD | Stevioside | RebF | Reb M |
|---|---|---|---|---|---|---|
| 3.89% | 1.11% | 0.02% | 93.72% | 0.30% | 0.03% | 0.93% |

<Experimental Example 15>

Extraction and solid-liquid separation were performed in the same manner as in Experimental Example 10. The proportion of each steviol glycoside per TSG in the dry leaf (% by mass) was measured in the same manner as in Experimental Example 10, and was as shown in the following Table.

[Table 45]

| Reb A | Reb B | Reb C | Reb D | Stevioside | Reb F | Reb M |
|---|---|---|---|---|---|---|
| 53. 6 | 1. 2 | 4. 2 | 20. 5 | 11. 2 | 1. 1 | 8.2 |

Steps of flocculation, purification with the resin, and evaporation concentration were performed in the same manner as in Experimental Example 10. The resulting steviol glycoside composition was spray dried, to obtain a roughly purified product. The proportion of each steviol glycoside per TSG (herein including eight kinds, RebA, RebB, RebC, RebD, stevioside, RebF, RebM, and RebN) in the roughly purified product after the spray drying (% by mass) is as follows. The proportion of TSG per roughly purified product was 95.75%.

[Table 46]

| RebA | RebB | RebC | RebD | Stevioside | RebF | RebM | RebN |
|---|---|---|---|---|---|---|---|
| 49.4 | 0.4 | 4.6 | 14.8 | 13.4 | 1.1 | 5.7 | 3.4 |

Crystallization was performed in the same manner as in Experimental Example 9 except that conditions in the following Table were adopted. The degree of supersaturation of RebA (10°C) was 5.7 and the degree of supersaturation of RebD (10°C) was 160.1.

[Table 47]

| Primary crystallization parameters | |
|---|---|
| Solvent | 90% by mass EtOH 1,538 ml |
| Amount of roughly purified product added | 179.79g |
| Liquid temperature (Start) | 55°C |
| Liquid temperature (End) | 10°C |
| Time | 7.5H |
| Stirring speed | 200rpm |
| Temperature profile | Controlled |
| Seed crystal (RebD) | Cs=0. 08 |

Secondary crystallization was performed in the same manner as in the primary crystallization except that conditions in the following Table were adopted.

[Table 48]

| Secondary crystallization parameters | |
|---|---|
| Amount of primary crystallized product added | About 42g |
| Solvent | 90% by mass EtOH 715 ml |
| Ratio of RebD to solvent | 3.4% by mass |
| Liquid temperature (Start) | 55°C |
| Liquid temperature (End) | 10°C |
| Time | 24H |
| Stirring speed | 200rpm |
| Temperature profile | Controlled |
| Seed crystal | - |

The purity and the degree of supersaturation of each component, and the yield ratio of RebA per unit step-the yield ratio of RebD per unit step were calculated in the same manner as in Experimental Examples 10 to 12. The results are shown in the following Tables. While a purity of more than 100% was shown in the following Tables, the reason for this was considered because the weight was increased due to the remaining solvent or there was any analysis error.

[Table 49]

| Purity of RebA before secondary crystallization (%) | Purity of RebD before secondary crystallization (%) | Purity of TSG before secondary crystallization (%) | Degree of supersaturation σ (RebA) at 10°C | Degree of supersaturation σ (RebD) at 10°C | σ (D)/ σ (A) | Purity of RebA after secondary crystallization (%) | Purity of RebD after secondary crystallization (%) | Yield ratio of RebD per unit step (%) |
|---|---|---|---|---|---|---|---|---|
| 21.8 | 55.1 | 112.8 | 0.6433 | 334.25 | 519.6 | 11.5 | 849 | 85.7 |

[Table 50]

| | Solvent | Amount of solvent (ml) | Seed crystal (mq) | Temperature (initial) | Temperature (end) | Time (H) | Purity of RebD (%) |
|---|---|---|---|---|---|---|---|
| First crystallization | 90% EtOH | 15386 | 1957 | 55 | 10 | 7.5 | 55.1 |
| Second crystallization | 90% EtOH | 715 | - | 55 | 10 | 24 | 85.0 |

<Experimental Example 16>

Extraction and solid-liquid separation were performed in the same manner as in Experimental Example 10. The proportion of each steviol glycoside per TSG in the dry leaf (% by mass) was measured in the same manner as in Experimental Example 10, and was as shown in the following Table.

[Table 51]

| Reb A | Reb B | Reb C | Reb D | Stevioside | Reb F | Reb M |
|---|---|---|---|---|---|---|
| 68.4 | 1.0 | 5. 4 | 9.2 | 7.8 | 1.5 | 6.6 |

Steps of flocculation, purification with the resin, and evaporation concentration were performed in the same manner as in Experimental Example 10. The resulting steviol glycoside composition was spray dried, to obtain a roughly purified product. The compositional ratio of the roughly purified product after the spray drying (% by mass) is as follows.

[Table 52]

| Reb A | Reb B | Reb C | Reb D | Stevioside | Reb F | Reb M | Others |
|---|---|---|---|---|---|---|---|
| 64. 8 | 0.6 | 5. 5 | 8. 1 | 9. 1 | 1.3 | 4. 7 | 6.0 |

Primary crystallization was performed in the same manner as in Experimental Example 9 except that conditions in the following Table were adopted.

[Table 53]

| Primary crystallization parameters | |
|---|---|
| Solvent | 90% by mass EtOH 1,117 ml |
| Ratio of RebD to solvent | 1.6% by mass |
| Liquid temperature (Start) | 55°C |
| Liquid temperature (End) | 10°C |
| Time | 7. 5H |
| Stirring speed | 350rpm |
| Temperature profile | Controlled |
| Seed crystal (RebD) | Cs=0. 08 |

Secondary crystallization was performed in the same manner as in the primary crystallization except that conditions in the following Table were adopted.

[Table 54]

| Secondary crystallization parameters | |
|---|---|
| Solvent | 90% by mass EtOH 715 ml |

(continued)

| Secondary crystallization parameters | |
|---|---|
| Amount of RebA in primary crystallized product added | 5.915g |
| Amount of RebD in primary crystallized product added | 14.442g |
| Liquid temperature (Start) | 55°C |
| Liquid temperature (End) | 10°C |
| Time | 24H |
| Stirring speed | 350rpm |
| Temperature profile | Controlled |
| Seed crystal (RebD) | Cs=0.08 |

The purity and the degree of supersaturation of each component, and the yield ratio of RebA per unit step-the yield ratio of RebD per unit step were calculated in the same manner as in Experimental Examples 10 to 12. The results are shown in the following Table. While a purity of more than 100% was shown in the following Tables, the reason for this was considered because the weight was increased due to the remaining solvent or there was any analysis error.

[Table 55]

| Purity of RebA before primary crystallization (% by mass) | Purity of RebD before primary crystallization (% by mass) | Purity of TSG before primary crystallization (% by mass) | Degree of supersaturation σ (RebA) before primary crystallization | Degree of supersaturation σ (RebD) before primary crystallization | σ (D) / σ (A) | Purity of RebA after primary crystallization (% by mass) | Purity of RebD after primary crystallization (% by mass) | Yield ratio of RebA per unit step in primary crystallization (%) | Yield ratio of RebD per unit step in primary crystallization (%) |
|---|---|---|---|---|---|---|---|---|---|
| 64.8 | 8.1 | 94 | 14.7 | 161.2 | 11.0 | 26.0 | 63.4 | 4.1 | 82.5 |

| Purity of RebA before secondary crystallization (%) | Purity of RebD before secondary crystallization (%) | Purity of TSG before secondary crystallization (%) | Degree of supersaturation σ (RebA) at 10°C | Degree of supersaturation σ (RebD) at 10°C | σ (D) / σ (A) | Purity of RebA after secondary crystallization (%) | Purity of RebD after secondary crystallization (%) | Yield ratio of RebD per unit step (%) |
|---|---|---|---|---|---|---|---|---|
| 26.0 | 63.4 | 109.5 | 0.00027 | 202.348 | 749437.0 | 4.0 | 92.2 | 97.7 |

[Table 56]

| | Solvent | Amount of solvent (ml) | Seed crystal (mg) | Temperature (initial) | Temperature (end) | Time (H) | Purity of RebD (%) |
|---|---|---|---|---|---|---|---|
| First crystallization | 90% EtOH | 1117.1 | 1240 | 55 | 10 | 7.5 | 63.4 |
| Second ciystallization | 90% EtOH | 715 | 1149 | 55 | 10 | 24 | 92.2 |

**Claims**

1. A method for producing a rebaudioside D-containing crystallized product by use of a roughly purified product obtained by roughly purifying an extract from a stevia plant, wherein

   a total steviol glycoside content of the roughly purified product is 50 to 95% by mass and the roughly purified product contains at least rebaudioside A and rebaudioside D,
   the method comprising:

   a step of mixing the roughly purified product in a solvent containing ethanol and having a methanol concentration of 1 mg/L or less, to adjust a solution for crystallization, and
   a step of cooling the solution for crystallization with stirring, to precipitate rebaudioside D.

2. The production method according to claim 1, wherein the total steviol glycoside corresponds to rebaudioside A, rebaudioside B, rebaudioside C, rebaudioside D, stevioside, rebaudioside F and rebaudioside M.

3. The production method according to claim 1 or 2, wherein a content of rebaudioside A is 5 to 70% by mass and a content of rebaudioside D is 2 to 70% by mass in the roughly purified product.

4. The production method according to any one of claims 1 to 3, wherein a concentration of the ethanol in the solvent is 99.9% by mass or less.

5. The production method according to any one of claims 1 to 4, wherein the solvent is kept at a temperature of 40 to 80°C in mixing of the roughly purified product.

6. The production method according to any one of claims 1 to 5, wherein rebaudioside D is used as a seed crystal.

7. The production method according to any one of claims 1 to 6, wherein the solution for crystallization is cooled to a temperature of 35°C or less with stirring, to precipitate rebaudioside D.

8. The production method according to any one of claims 1 to 7, wherein the solution for crystallization is cooled at a rate of 0.002 to 1.37°C/min with stirring, to precipitate rebaudioside D.

9. The production method according to any one of claims 1 to 8, wherein the solution for crystallization is cooled for a period of 1 to 48 hours with stirring, to precipitate rebaudioside D.

10. The production method according to any one of claims 1 to 9, wherein the roughly purified product is obtained by a method comprising

an extraction step of extracting a dry leaf of a stevia plant with a solvent to obtain an extract,
a solid-liquid separation step of subjecting the extract to a solid-liquid separation treatment to obtain a clarified liquid,
an flocculation step of adding a coagulant to the clarified liquid for flocculation, to obtain a treatment liquid,
a resin purification step of treating the treatment liquid with a hydrophobic porous resin, and
a concentration step of concentrating a solution after purification with the resin.

11. The production method according to any one of claims 1 to 10, further comprising a step of separating rebaudioside D precipitated and a liquid phase, and drying rebaudioside D separated.

12. The production method according to any one of claims 1 to 10, further comprising

a step of separating and drying rebaudioside D precipitated, to obtain a primary crystallized product,
a step of mixing the primary crystallized product in a solvent containing ethanol and having a methanol concentration of 1 mg/L or less, to adjust a primary crystallized product-dissolved liquid,
a step of cooling the primary crystallized product-dissolved liquid with stirring, to precipitate rebaudioside D, and
a step of separating and drying rebaudioside D precipitated.

13. The production method according to any one of claims 1 to 12, wherein a methanol content of a rebaudioside D-containing crystallized product is less than 10 ppm.

14. The production method according to any one of claims 1 to 13, wherein a ratio MeOH/EtOH of methanol to ethanol contained in a rebaudioside D-containing crystallized product is 0.00010 to 0.00080.

15. The production method according to any one of claims 1 to 14, wherein a ratio of rebaudioside D to the total steviol glycoside in a rebaudioside D-containing crystallized product is 35 to 95% by mass.

16. The production method according to claim 15, wherein a ratio of rebaudioside A to the total steviol glycoside in the rebaudioside D-containing crystallized product is 10 to 50% by mass.

17. The production method according to any one of claims 1 to 16, wherein a ratio of rebaudioside D crystallized, to rebaudioside D contained in a roughly purified product, in the case of single crystallization, is 70 to 99% by mass.

18. The production method according to any one of claims 1 to 17, wherein, in the step of adjusting the solution for crystallization, the roughly purified product is mixed in the solvent containing ethanol such that a degree of supersaturation of rebaudioside D at 10°C is 10 or more and a degree of supersaturation of rebaudioside A at 10°C is 18 or less.

19. A rebaudioside D-containing crystallized product, having a methanol content of less than 10 ppm.

20. The rebaudioside D-containing crystallized product according to claim 19, wherein a ratio MeOH/EtOH of methanol to ethanol contained is 0.00010 to 0.00080.

21. A rebaudioside D-containing crystallized product produced by the production method according to any one of claims 1 to 18.

22. A food or drink product comprising the rebaudioside D-containing crystallized product according to any one of claims 19 to 21.

23. The food or drink product according to claim 22, which is a drink.

## Fig. 1

Fig. 2

Fig. 3

Fig. 4

Fig. 5

Fig. 6

Fig. 7

Fig. 8

Fig. 9

Fig. 10

Fig. 11

## Fig. 12

## Fig. 13

| | INTERNATIONAL SEARCH REPORT | International application No. |
| --- | --- | --- |
| | | PCT/JP2020/040975 |

**A. CLASSIFICATION OF SUBJECT MATTER**
B01D 9/02(2006.01)i; C07H 15/256(2006.01)i; A23L 27/00(2016.01)i; A23L 2/60(2006.01)i
FI:     C07H15/256 A; A23L27/00 101A; A23L2/60; B01D9/02 601H; B01D9/02 602B; B01D9/02 603B; B01D9/02 604; B01D9/02 608A; B01D9/02 625A; B01D9/02 620; B01D9/02 625E

According to International Patent Classification (IPC) or to both national classification and IPC

**B. FIELDS SEARCHED**

Minimum documentation searched (classification system followed by classification symbols)
B01D9/02; C07H15/256; A23L27/00; A23L2/60

Documentation searched other than minimum documentation to the extent that such documents are included in the fields searched
Published examined utility model applications of Japan        1922–1996
Published unexamined utility model applications of Japan      1971–2020
Registered utility model specifications of Japan              1996–2020
Published registered utility model applications of Japan      1994–2020

Electronic data base consulted during the international search (name of data base and, where practicable, search terms used)
CAplus/REGISTRY (STN)

**C. DOCUMENTS CONSIDERED TO BE RELEVANT**

| Category* | Citation of document, with indication, where appropriate, of the relevant passages | Relevant to claim No. |
| --- | --- | --- |
| X | JP 2018-530326 A (THE COCA-COLA COMPANY) 18 October 2018 (2018-10-18) claims, examples | 1, 3-23 |
| Y | claims, examples | 2 |
| X | JP 2013-507914 A (PURECIRCLE SDN BHD) 07 March 2013 (2013-03-07) claims, examples 1-8, 10-13 | 1, 3-9, 11-23 |
| Y | claims, examples 1-8, 10-13 | 2 |
| Y | paragraphs [0026]-[0099] | 10 |
| X | WO 2017/218072 A1 (PURECIRCLE USA INC.) 21 December 2017 (2017-12-21) claims, examples | 1-9, 11-23 |
| Y | claims, examples | 10 |
| Y | tables 1-2 | 2 |

☒ Further documents are listed in the continuation of Box C.    ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 18 December 2020 (18.12.2020) | 12 January 2021 (12.01.2021) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2020/040975

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 1-131191 A (GIOVANETTO, Roger H.) 24 May 1989 (1989-05-24) claims, example | 10 |
| Y | US 2006/0142555 A1 (COUNCIL OF SCIENTIFIC AND INDUSTRIAL RESEARCH) 29 June 2006 (2006-06-29) claims, examples | 10 |

Form PCT/ISA/210 (continuation of second sheet) (January 2015)

**INTERNATIONAL SEARCH REPORT**
Information on patent family members

International application No.

PCT/JP2020/040975

| Patent Documents referred in the Report | Publication Date | Patent Family | Publication Date |
|---|---|---|---|
| JP 2018-530326 A | 18 Oct. 2018 | US 2018/0263269 A1<br>claims, examples<br>WO 2017/059414 A1<br>EP 3355720 A1<br>CA 3000719 A<br>AU 2016329208 A<br>CN 108289487 A<br>MX 2018004068 A<br>HK 1258382 A | |
| JP 2013-507914 A | 07 Mar. 2013 | US 2011/0092684 A1<br>claims, paragraphs<br>[0025]-[0104],<br>examples<br>WO 2011/046423 A1<br>EP 2358730 A1<br>AR 78647 A<br>CA 2775732 A<br>KR 10-2012-0065961 A<br>MX 2012003686 A<br>RU 2011115775 A<br>ES 2457641 T<br>ES 2661539 T<br>BR PI1005384 A<br>PL 2708548 T<br>CA 3015571 A | |
| WO 2017/218072 A1 | 21 Dec. 2017 | JP 2019-518065 A<br>claims, examples<br>US 2019/0133167 A1<br>EP 3468385 A1<br>CN 109640693 A<br>BR 112018076109 A<br>MX 2018015591 A | |
| JP 1-131191 A | 24 May 1989 | US 4892938 A<br>claims, examples<br>EP 302948 A3<br>SU 1834646 A<br>NO 173913 B<br>NZ 225471 A<br>ES 2046979 T<br>DD 273442 A<br>DK 406188 A<br>IE 63706 B<br>ZA 8805176 A<br>AT 97910 T<br>CA 1310963 A<br>IN 171592 A<br>CN 1030762 A<br>AU 1921988 A<br>IN 171592 B | |
| US 2006/0142555 A1 | 29 Jun. 2006 | (Family: none) | |

Form PCT/ISA/210 (patent family annex) (January 2015)

**EP 4 052 772 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013507914 A
- JP 2019518065 A
- WO 2019074089 A